# EUROPEAN PATENT APPLICATION

(11) **EP 4 491 630 A1**
(43) Date of publication of application: **15.01.2025**
(21) Application number: 23386059.2
(22) Date of filing: 12.07.2023
(51) Int. Cl.: C07K 14/725, A61K 39/00, A61P 35/00, C07K 14/705, C12N 9/12

(54) **T-CELL RECEPTOR FOR ACUTE MYELOID LEUKEMIA (AML)**

(71) Applicant: Universitetet I Oslo, 0316 Oslo (NO); Oslo Universitetssykehus HF, 0424 Oslo (NO)
(72) Inventor: Olweus, Johanna, 1359 Eiksmarka (NO); Giannakopoulou, Eirini, 0485 Oslo (NO)
(74) Representative: Onsagers AS

(57) **Abstract**

The present disclosure provides proteins comprising a targeting unit able to recognize epitopes comprising the D835Y when presented by HLA on the surface of cells. Cells expressing a TCR comprising the targeting unit mediated highly specific and efficient killing of primary AML cells harboring the FLT3^{D835Y} mutation *in vitro*, and *in vivo.*

## Description

### Field

The present disclosure concerns antigen binding proteins based on sequences from T-cell receptors (TCRs). The proteins disclosed herein, and cells expressing them, may be used in cancer therapy.

### Background

The only curative therapeutic option for many AML patients today is allogeneic hematopoietic stem cell transplantation (allo-HSCT). Allo-HSCT remains, however, associated with high relapse rates as well as transplant-related morbidity and mortality resulting from donor T cells attacking healthy recipient cells, causing severe graft-versus-host disease (GVHD), and often a suitable donor cannot be identified. This has prompted development of alternative cellular therapies. Successful targeting of the lymphoid-specific molecule CD19 in chimeric antigen receptor (CAR) T-cell therapy of acute lymphocytic leukemia has led to attempts at also directing CARs to myeloid cell surface antigens, including CD33, CD123 and FLT3, overexpressed on AML cells. However, these molecules are also highly expressed on normal myeloid progenitor cells and even on hematopoietic stem cells, representing a major challenge. Thus, CAR T therapies targeting these molecules are associated with toxicities and a need for transplantation to rescue normal hematopoiesis after a short period of treatment. It is also unclear whether all AML propagating cells express these antigens, and therefore whether the targeting has curative potential.

Recurrent driver mutations in FMS-like tyrosine kinase 3 (FLT3) occur in approximately 1/3 of de novo AML cases, as internal tandem duplications (ITD) in the juxtamembrane domain or point mutations in the activation loop of the tyrosine kinase domain (TKD), which are screened for in routine diagnostics of AML. Although in most cases FLT3 mutations are secondary events in leukemogenesis, they are associated with accelerated clonal expansion and disease progression, and treatment with the tyrosine kinase inhibitor (TKI) midostaurin in patients with FLT3 mutations receiving standard induction chemotherapy does significantly increase long-term survival. This indicates that FLT3 mutated AML clones have a survival advantage during standard therapy, expanding to drive relapse unless effectively eradicated, but the efficacy of TKIs in eliminating FLT3 mutated clones remains variable.

Because of varying length, ITDs do not encode shared neoantigens. Mutations in the D835 position represent the most frequent point mutation in FLT3 (7-10% of AML patients), with aspartic acid (D) to tyrosine (Y) being by far the most common amino acid substitution, although other substitutions also can occur. The effect of TKI's on the D835Y mutation (FLT3^{D835Y}) is limited by this mutation conferring primary and secondary resistance to 1st and 2nd generation type II tyrosine-kinase inhibitors.

### Summary

Because the D835Y substitution in FLT3 is located intracellularly, an epitope comprising it cannot be targeted by conventional antibodies or CARs. However, the present disclosure provides proteins comprising a targeting unit able to recognize epitopes comprising the D835Y when presented by HLA on the surface of cells. Cells expressing a TCR comprising the targeting unit mediated highly specific and efficient killing of primary AML cells harboring the FLT3^{D835Y} mutation *in vitro,* and *in vivo.* The TCR (TCR^{FLT3D/Y}) is restricted by HLA-A*02:01 (hereafter HLA-A2), expressed in approximately 50% of Caucasians. T-cells redirected with TCR^{FLT3D/Y} (TCR^{FLT3D/Y} cells) specifically and efficiently eliminate primary human AML cells harboring FLT3^{D835Y} *in vitro* and *in vivo,* while sparing cells expressing wild type (WT) FLT3.

In a first aspect, the present disclosure provides a protein for specific binding to a human leukocyte antigen (HLA) type A2 presenting an HLA-class 1 peptide comprising the sequence YIMSDSNYV (SEQ ID NO: 1),
wherein the protein comprises an α-chain variable domain and a β-chain variable domain, which together form an antigen binding unit;
wherein the α-chain variable domain comprises three complementarity-determining regions (CDRs); CDR1, CDR2 and CDR3 which respectively comprise the amino acid sequences SEQ ID NO: 2, 3 and 4,
   and
wherein the β-chain variable domain comprises three CDRs; CDR1, CDR2 and CDR3 which respectively comprise the amino acid sequences SEQ ID NO: 5, 6 and 7.

In one embodiment of the first aspect,
the α-chain variable domain comprises the amino acid sequence set forth in SEQ ID NO: 8, or an amino acid sequence having at least 90 % sequence identity thereto; and
the β-chain variable domain comprises the amino acid sequence set forth in SEQ ID NO: 9, or an amino acid sequence having at least 90 % sequence identity thereto.

In a second embodiment of the first aspect,
the α-chain variable domain is fused to an extracellular α-chain constant domain and the β-chain variable domain is fused to an extracellular β-chain constant domain. Said extracellular α-chain constant domain may be human, murine or
synthetic. Said extracellular β-chain constant domain may be human, murine or synthetic.

In a third embodiment of the first aspect,
the extracellular α-chain constant domain is represented by SEQ ID NO: 10 and the extracellular β-chain constant domain represented by SEQ ID NO: 11.

In a fourth embodiment of the first aspect,
the extracellular α-chain constant domain is fused to a transmembrane domain.

In a fifth embodiment of the first aspect, the extracellular β-chain constant domain is fused to a transmembrane domain.

In a sixth embodiment of the first aspect,
each of the extracellular α-chain constant domain and the extracellular β-chain constant domain are fused to a transmembrane domain.

In a seventh embodiment of the first aspect,
the transmembrane domain is fused to an intracellular signaling domain.

In an eighth embodiment of the first aspect,
the protein comprises a full-length α-chain and/or a full-length β-chain.

In a nineth embodiment of the first aspect,
the protein comprises a full-length α-chain represented by SEQ ID NO: 12 and/or a full-length β-chain represented by SEQ ID NO: 13, or alternatively, a protein according to claim 1 comprising a full-length α-chain represented by SEQ ID NO: 14 and/or a full-length β-chain represented by SEQ ID NO: 15.

In a second aspect, the present disclosure provides one or more recombinant nucleic acid molecules which alone or together encode a protein according to the first aspect.

In a third aspect, the present disclosure provides one or more cDNA molecules encoding a protein according to the first aspect.

In a fourth aspect, the present disclosure provides an immune effector cell transduced with a nucleic acid according to the second aspect.

In a fifth aspect, the present disclosure provides an immune effector cell expressing a protein according to the first aspect.

In a sixth aspect, the present disclosure provides an immune effector cell according to the fourth or fifth aspect, wherein said cell is a T-helper cell, a cytotoxic T-cell or a natural killer cell.

In a seventh aspect, the present disclosure provides a pharmaceutical composition comprising the proteins according to the first aspect, the nucleic acid according to second aspect, or a cell according to the fourth or fifth aspect.

In an eighth aspect, the present disclosure provides a pharmaceutical composition for intravenous administration comprising T-cells expressing a protein according to any one of the first aspect in their cell membrane.

In a nineth aspect, the present disclosure provides a method for treatment of acute myeloid leukemia (AML)
comprising the steps of
a) providing a biological sample comprising cancer cells from a subject diagnosed with AML,
b) performing an assay for detecting the D835Y mutation in FLT3 in the biological sample,
c) performing HLA-typing of the subject, and
d) administering the pharmaceutical composition as defined in the eighth aspect via intravenous infusion to the subject if the assay in b) is positive and the subject is HLA-A*02:01 positive.

The steps b) and c) in the nineth aspect, can be performed in any order, and step c) may be performed at any time.

Accordingly, the present disclosure provides a method for treatment of acute myeloid leukemia (AML)
comprising the steps of
a) providing a biological sample comprising cancer cells from a subject diagnosed with AML,
b) performing an assay for detecting the D835Y mutation in FLT3 in the biological sample,
c) administering the pharmaceutical composition as defined in the eighth aspect via intravenous infusion to the subject if the assay in b) is positive and the subject is HLA-A*02:01 positive.

### Brief description of the figures

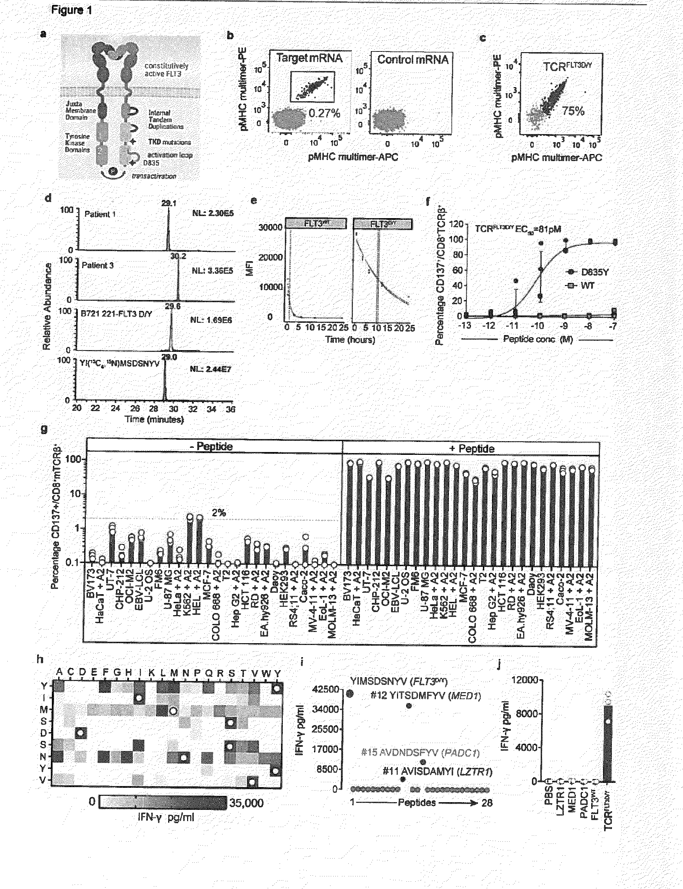

**Figure 1****: TCR^{FLT3D/Y} cells specifically recognize mutated peptide with high sensitivity in an HLA-A*02:01-restricted manner and do not show off-target reactivity**
   **(a)** Schematic illustration of the structure of FLT3, including the sites of the most common activating mutations. **(b)** CD8⁺ T cells stained with pMHC multimers complexed with the FLT3^{D/Y} peptide (APC and PE) following co-cultures of HLA-A2^{pos} naive CD8⁺ T cells with autologous HLA-A2^{pos} moDCs transfected with target or control mRNA. **(c)** CD8⁺ T cells transduced with the FLT3^{D/Y} TCR stained with FLT3^{D/Y} pMHC multimers. **(d)** Parallel Reaction Monitoring (PRM) analysis, targeting the presence of FLT3-D835Y peptide (m/z=1091.43891+) in primary AML cells from two patient samples and the B721.221 cell line transduced to express D835Y and HLA-A*02:01. The ion chromatograms were extracted using the Precursor→b2, b3, b4, b5, b6, b7 and b8 fragment ion transactions. **(e)** Off-rates for FLT3^{WT} or FLT3^{D/Y} peptide binding to HLA-A2 coupled to fluorescent particles as measured by flow cytometry, with calculated half-life indicated by vertical lines (one line per experiment). Dots represent MFI values corresponding to staining of intact pMHC complexes at indicated timepoints (hours). Results are from three independent experiments with each circle representing one replicate for each experiment. **(f)** Activation of TCR^{FLT3D/Y} cells measured as upregulation of CD137 after co-incubation with K562 cells pulsed with indicated peptide concentrations. EC₅₀ = half maximal effective concentration. Data points are from 4 TCR-transduced donors in three independent experiments with each circle representing the mean of three technical replicates for each donor, and are shown as mean ± s.e.m. **(g)** Activation of CD8⁺ TCR^{FLT3D/Y} cells following co-culture with HLA-A2^{pos} cell lines of different tissue origins in absence or presence of the FLT3^{D/Y} peptide (10nM). Results are from one experiment representative of 2-4 performed with different T-cell donors, data points represent three technical replicates. Connecting lines in F and bars in G show mean. Data are normalized to transduction efficiency. Dashed line indicates the highest level of activation by cell lines alone (2%). **(h)** Heat map showing IFN-γ produced by TCR^{FLT3D/Y} cells co-incubated with K562 cells loaded with peptides from the mimotope library (10nM). Amino acids of the FLT3^{D/Y} peptide are indicated by white circles. IFN-γ concentration range for positive reactions was 5000 - 35,000 pg/mL. **(i-j)** IFN-γ production of TCR^{FLT3D/Y} cells co-incubated with K562 cells (i) pulsed with the peptides that were predicted as potentially cross-reactive from the in silico search, or (j) transfected with mRNA constructs encoding 30-32mer peptides with the candidate cross-reactive peptide inducing reactivity (shown in (i)) in the middle, flanked by its naturally occurring sequence, or transfected with mRNA encoding the FLT3^{D/Y} epitope or FLT3^{WT}. Data in h-j are from one experiment out of two performed, and individual data points represent either one (h,i) or three (j) technical replicates.

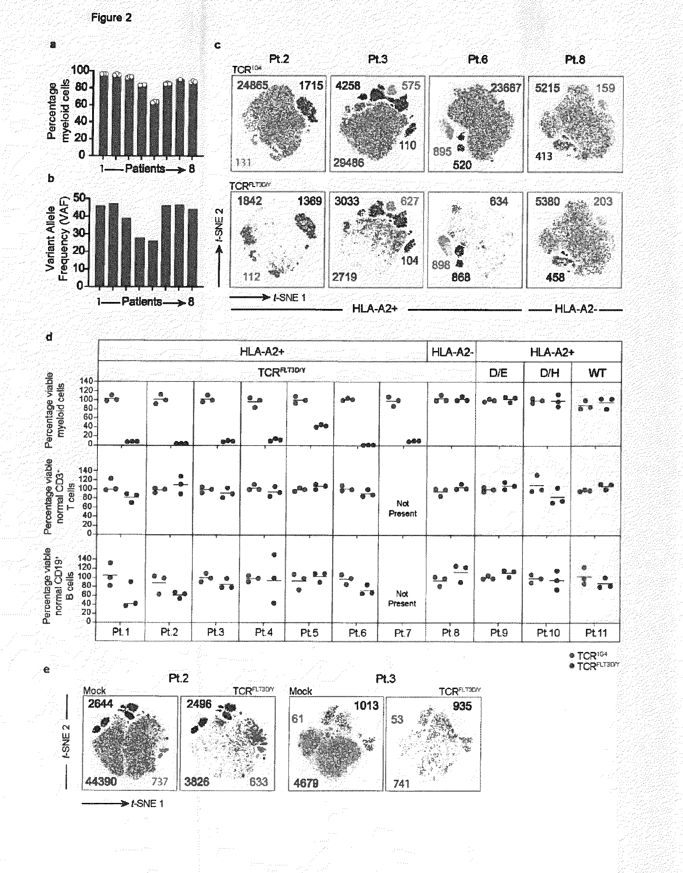

**Figure 2****: TCR^{FLT3D/Y} cells efficiently kill primary AML cells harboring the FLT3 D835Y mutation *in vitro* but spare normal lymphoid cells**
   **(a)** Percentage myeloid cells for AML patients (Pt) 1-8 of live leukocytes. **(b)** Peripheral blood (PB) or bone marrow (BM) FLT3^{D/Y} variant allele frequency (VAF) for patients 1-8 as determined by next-generation sequencing. **(c)** Representative *t*-distributed stochastic neighbor embedding (*t*-SNE) plots showing live primary myeloid cells (CD3⁻CD19⁻CD20⁻ events) in red, T cells (CD3⁺) in blue, B cells (CD19⁺CD20⁺) in orange and normal CD34⁺lin⁻ progenitor cells in green, from three representative HLA-A2^{pos} FLT3^{D/Y} AML patients (Pt 2, 3 and 6) and one HLA-A2^{neg} FLT3^{D/Y} case (Pt 8), following 72 h of co-culture with TCR^{1G4} (negative control, top row) or TCR^{FLT3D/Y} cells (E:T ratio = 1:2), as quantified by flow cytometry. TCR-transduced cells were excluded from analysis as CTV positive events. **(d)** Diagnostic samples from 11 AML patients with FLT3^{D/Y} (Pt 1-8), FLT3^{D/E} (Pt 9) or FLT3^{D/H} (Pt 10) mutation, or FLT3^{WT} (Pt 11) (all HLA-A2^{pos} except Pt 8), analyzed as described in (c). Each dot represents the fraction of live myeloid cells, B cells or T cells, after co-culture with TCR^{FLT3D/Y} cells (purple), in percent of mean of corresponding numbers in cultures treated with TCR^{1G4} cells (blue). Data points represent 3 technical replicates and horizontal lines show mean. Data shown are from one experiment representative of 2-4 performed for each patient sample (n=1 only for pt 7). **(e)** *t*-SNE plots of PB diagnostic samples from AML patients 2 and 3 showing live myeloid, T and B cells (color coded as in Fig. 2c) after 72 h co-culture with autologous T cells transduced with TCR^{FLT3D/Y} or Mock. Inset numbers in c and e denote absolute event counts of indicated cell populations.

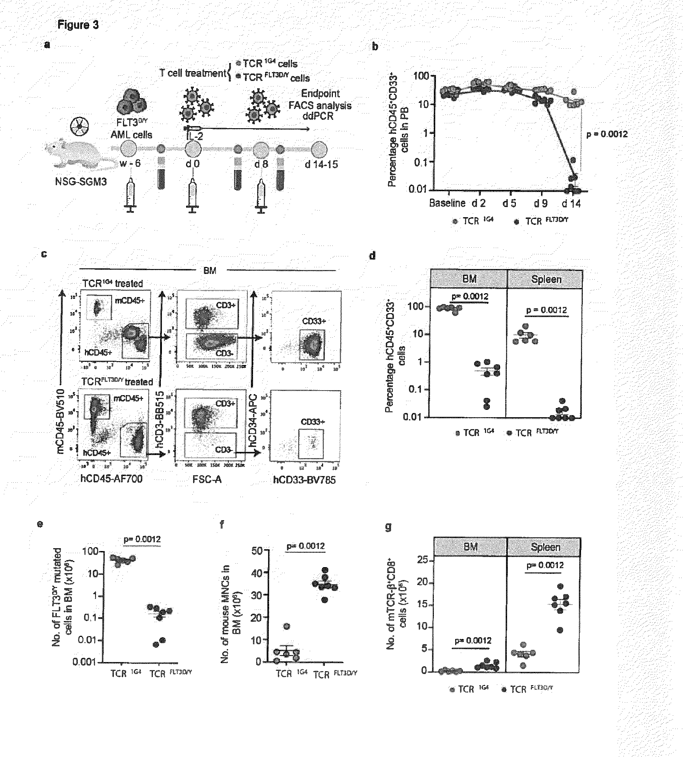

**Figure 3****: TCR^{FLT3D/Y} cells efficiently target primary AML in mice with high leukemic burden**
   **(a)** Schematic overview of the patient-derived xenograft (PDX) *in vivo* model with FLT3^{D/Y} mutated primary AML cells from patient 7. **(b)** Percentage of human hCD45⁺CD33⁺ cells in PB at baseline (1 day prior to T cell infusion) and on indicated days after infusion of TCR^{1G4} *(n* = 6) or TCR^{FLT3D/Y} *(n* = 7) cells. Numbers adjusted for hCD3⁺ T cells. **(c)** Representative FACS plots of viable single BM mononuclear cells (MNCs) from TCR^{1G4} (top) and TCR^{FLT3D/Y} (bottom) cell-treated NSG-SGM3 mice stably engrafted with primary AML FLT3^{D/Y} cells from patient 7. **(d)** Percentage of hCD45⁺CD33⁺ cells in BM and spleen at terminal analysis 15 days post T cell infusion. Numbers adjusted for hCD3⁺ T cells. **(e)** Number of FLT3^{D/Y} mutated BM hCD45⁺CD3⁻ cells determined by ddPCR. **(f-g)** Number of mouse (m) CD45⁺ cells in BM (f) and mTCR-β⁺CD8⁺ cells in BM and spleen (g) at endpoint. All data are presented as mean ± s.e.m. and are generated from one experiment. Each dot represents one mouse and statistical analysis was performed with two-tailed Mann-Whitney test. P values are shown and p < 0.05 was considered statistically significant.

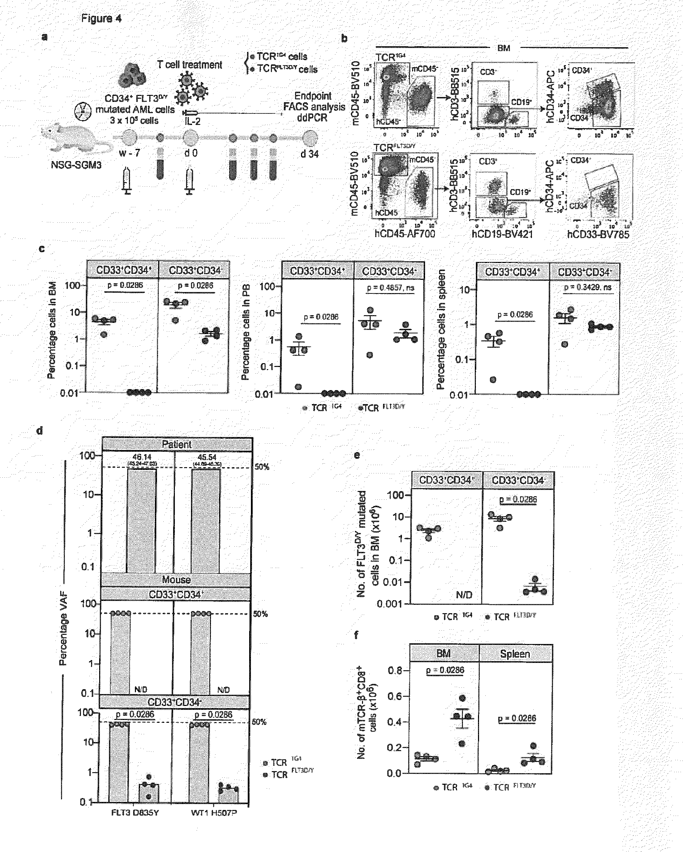

**Figure 4****: TCR^{FLT3D/Y} cells eliminate primary CD34+ AML *in vivo***
   **(a)** Schematic overview of the PDX *in vivo* model with FLT3^{D/Y} mutated primary AML cells from patient 1. **(b)** Representative FACS plots of BM from TCR^{1G4} (top) and TCR^{FLT3D/Y} (bottom) cell-treated NSG-SGM3 mice stably engrafted with primary AML FLT3^{D/Y} cells from patient 1. Equivalent gating was also used for PB and spleen. **(c)** Percentage of hCD45⁺CD33⁺CD34⁺ and hCD45⁺CD33⁺CD34⁻ cells in BM, PB and spleen at endpoint (day 34 post T cell infusion) of TCR^{1G4} cell (*n* = 4) or TCR^{FLT3D/Y} cell *(n* = 4) treated mice. Numbers adjusted for hCD3⁺ T cells. **(d)** Percentage VAF determined by ddPCR of FLT3^{D835Y} and WT1^{H507P} driver mutations in primary BM cells from patient 1 (top), and TCR cell treated mice hCD45⁺CD33⁺CD34⁺ (middle) and hCD45⁺CD33⁺CD34⁻ (bottom). N/D not analyzed due to insufficient hCD45⁺CD33⁺CD34⁺ cells. Numbers show VAF and 95% confidence interval. Dotted line at 50% indicates 100% clonality unless loss of heterozygocity. No significant differences in VAFs of FLT3^{D835Y} and the WT1^{H507P} mutations were observed. **(e)** Number of FLT3^{D835Y} mutated hCD45⁺CD33⁺CD34⁺ and hCD45⁺CD33⁺CD34⁻cells in BM as determined by ddPCR. N/D not detected due to lack of hCD45⁺CD33⁺CD34⁺ cells. **(f)** Number of mTCR-β⁺CD8⁺ cells in BM and spleen at endpoint. All data are presented as mean ± s.e.m. from terminal analysis 34 days post T cell infusion from one experiment in which each dot represents one mouse and statistical analysis was performed with two-tailed Mann-Whitney test. *P* values are shown and *p* < 0.05 was considered statistically significant.

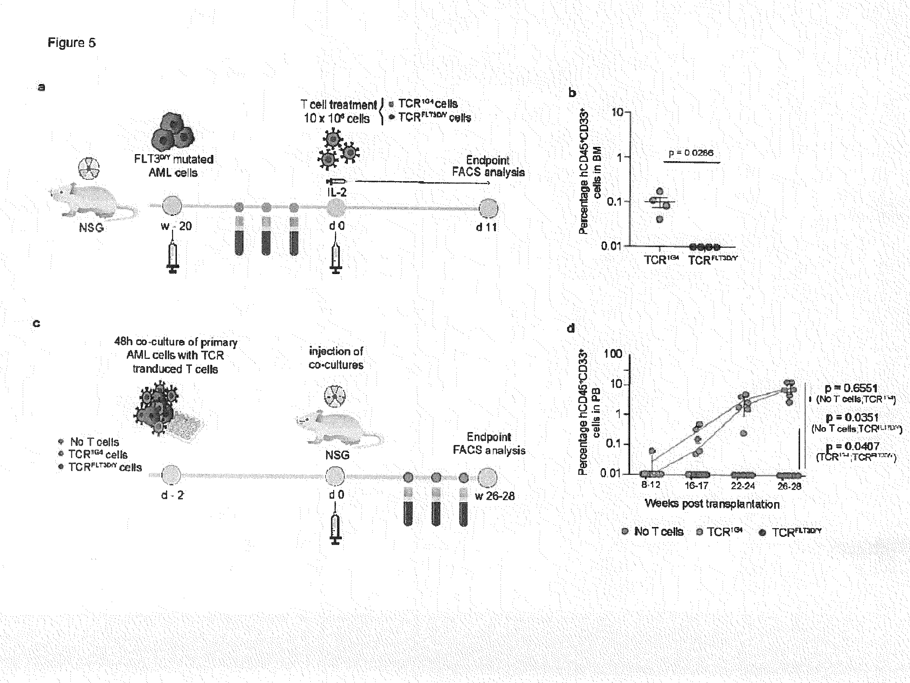

**Figure 5****: TCR^{FLT3D/Y} cells efficiently kill primary AML in a minimal residual disease setting and eliminate leukemia-propagating cells**
   **(a)** Schematic overview of the minimal residual disease (MRD) PDX *in vivo* model with FLT3^{D/Y} mutated primary AML cells from patient 1. **(b)** Percentage of hCD45⁺CD33⁺ cells in BM of NSG mice engrafted with low levels of AML after treatment with TCR^{1G4} *(n* = 4) or TCR^{FLT3D/Y} *(n* = 4) cells 11 days after T cell infusion. Numbers adjusted for hCD3⁺ T cells. Data are presented as mean ± s.e.m. and are generated from one experiment. Each dot represents one mouse and statistical analysis was performed with two-tailed Mann-Whitney test. **(c)** Schematic overview of the PDX *in vivo* model with FLT3^{D/Y} mutated primary AML cells from patient 1 after *in vitro* targeting with TCR^{1G4} or TCR^{FLT3D/Y} cells. **(d)** Percentage of hCD45⁺CD33⁺ cells in PB at indicated weeks after transplantation of primary AML cells from patient 1 following 48h co-culture without T cells *(n* = 3), or with TCR^{1G4} *(n* = 3) or TCR^{FLT3D/Y} (*n* = 5) cells. Data are presented as mean ± s.e.m. and are generated from two independent experiments. Each dot represents one mouse and statistical analysis was performed by multilevel linear regression using the R package "lmerTest" (further described in methods). *P* values are shown and *p* < 0.05 was considered statistically significant.

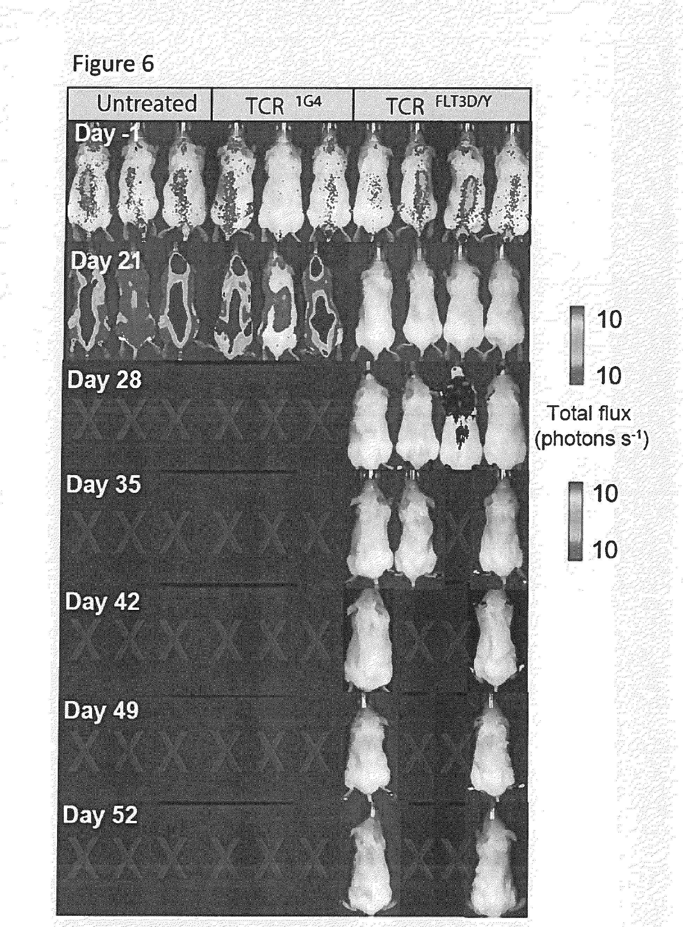

**Figure 6****: TCRFLT3D/Y cells efficiently target leukemia in a xenograft mouse model**
   BLI of BV173^{D835Y} engrafted leukemic cells in mice, one day before, and 21, 28, 35, 42, 49 and 52 days after treatment with TCR^{1G4} or TCR^{FLT3D/Y} cells or left untreated. One mouse relapsed d28 and was found dead on d32, while another was sacrificed on d41 after T cell treatment due to suspected GVHD, following negative BLI and PB flow cytometry analysis 6 days earlier, indicating that death was not related to leukemia.

### Detailed description

Neoantigens represent an attractive group of targets in cancer immunotherapy, as they are tumor-specific and can be recognized by T cells as foreign in context of MHC. Neoantigenic burden is an important determinant of clinical success upon checkpoint inhibition, and case reports have demonstrated that neoantigen-reactive T cells can mediate clinical responses. This has brought hope that T cells genetically modified to express TCRs derived from neoantigen-reactive T cells could provide efficient adoptive cell therapy. Patient T cells do, however, spontaneously recognize only 1-2% of candidate neoantigens predicted to be expressed and presented on HLA in solid cancer. As the large majority is unique to the individual patient, therapeutic targeting of neoantigens generally becomes a highly personalized effort. In addition to being resource-demanding, such a strategy might not benefit patients in time. In contrast, public neoantigens derived from recurrent oncogenic mutations have the advantage that a single TCR with off-the-shelf availability could target larger patient groups. Although shared mutations resulting in neoantigens presented on frequently expressed HLA alleles are rare in solid cancer, promising results were recently shown with T cells engineered to express a TCR targeting mutant KRAS in a patient with pancreatic adenocarcinoma, and mutant p53 in a breast cancer patient.

The present disclosure provides a TCR recognizing an epitope encoded by the recurrent driver mutation D835Y in the tyrosine-kinase domain of FLT3 in AML presented on the prevalent HLA-A2 allele. This TCR, selected from healthy donor T-cell repertoires, mediated highly specific and efficient killing of primary AML cells harboring the FLT3^{D835Y} mutation *in vitro,* and *in vivo.* Different clinically relevant aspects of therapeutic effects were demonstrated in different *in vivo* mouse PDX models. This included an almost complete removal of mutated CD34- AML in Model 1 with high engraftment, and complete removal of CD34+ AML in Model 2 with lower levels of reconstitution. In a third PDX model, the TCR^{FLT3D/Y} cells rejected patient AML cells in a setting resembling MRD. TCR^{FLT3D/Y} cells thus efficiently eliminated leukemia with both high and low disease burden. AML cells that are CD34⁺ have previously been shown to propagate leukemia *in vivo.* Here, we demonstrated in a fourth PDX model followed for seven months after AML injection, that TCR^{FLT3D/Y} cells *in vitro* can specifically and efficiently target and kill CD34⁺ FLT3^{D/Y} mutated AML cells. Studies in humans would, however, be required to confirm that the TCR^{FLT3D/Y} cells could achieve this also *in vivo.* Taken together, this suggests that a therapeutic TCR targeting a single, shared neoantigen has the potential of eliminating the reservoir of leukemia stem cells in AML.

The prospect of treating large patient groups with cytotoxic T cells expressing a single TCR targeting public neoantigens expressed by prevalent HLA molecules has emerged as an attractive therapeutic possibility. However, although a large number of recurrent hotspot mutations are known, it has proven difficult to identify HLA-bound peptides from tumor samples by immunopeptidomics. One reason might be that driver mutations that are poorly presented to the immune system provide a survival advantage. Another possibility is that mass spectrometry has insufficient sensitivity for detection of many neoantigenic peptides. We were, however, able to identify the FLT3^{D/Y} neoepitope in two AML patient samples by targeted mass spectrometry, starting with a large number of leukemia cells. T cells are, on the other hand, the most sensitive tools available for detection of peptide-MHC complexes. Our technology utilizing mRNA-transduced dendritic cells to prime naive T cells from healthy donors provides means to identify neoepitopes and reactive TCRs in a single assay. Presentation of the FLT3^{D/Y} neoepitope on leukemic cells was confirmed by efficient killing of primary HLA-A2^{pos} AML cells expressing D835Y, but not the less frequent D835E or D835H mutations or WT FLT3, by TCR^{FLT3D/Y} cells, further demonstrating TCR specificity.

To date, most efforts to isolate neoantigen-specific TCRs employ strategies whereby memory T cell responses in patient material are interrogated. Identification of TCRs with sufficient affinity is essential for clinical impact. A hurdle might be induction of T-cell tolerance due to long-standing co-evolution between cancer and the immune system in absence of inflammation, and lack of sufficient priming. This might contribute to the low spontaneous reactivity that tumor-infiltrating T cells show to the large repertoire of predicted neoantigens in melanoma. In support of this, immune responses to neoantigen vaccines seem to be dominated by *de novo* responses induced in naive T cells. The possibility to induce neoantigen-specific responses from naive patient-derived T cells is, however, limited by the amount of blood that can be harvested and by T cell repertoires that might be reduced by foregoing therapy. In an earlier study we showed that healthy donor-derived T cells recognized 5-fold more neoantigens than tumor-infiltrating lymphocytes (TILs) of melanoma patients. Here, we used the same approach to identify the TCR^{FLT3D/Y}, which recognizes picomolar peptide concentrations and mediates efficient cytotoxicity on primary AML cells *in vitro* and *in vivo.* High antigen sensitivity, indicated by EC₅₀ values below 10nM, appears to be an important factor for TCRs in effectively killing cancer cells, as previously demonstrated by us and others, Similarly, Foy *et al.,* in their publication in Nature, discussed the potential association between low EC₅₀ values observed for personalized neoantigen-reactive TCRs in their study and the restricted clinical efficacy observed .

We primed T cells from 16 healthy donors to identify T cells recognizing the YIMSDSNYV peptide presented by HLA-A*02:01. This indicates low immunogenicity of the peptide, consistent with lack of recognition by the investigated AML patient T cells, although a limited number of cells were screened for reactivity. Taken together, this demonstrates the advantage of accessing large, healthy donor T-cell repertoires. We previously demonstrated that pHLA stability is an important predictor of neoantigen immunogenicity. Here, we showed that the off rate for the FLT3⁸³⁵⁻⁸⁴³ peptide bound to HLA-A2 was significantly slower when substituting the WT amino acid D in position one with a Y. This is consistent with other studies showing that an increased stability of the neoantigen relative to the corresponding WT peptide leads to sustained antigen presentation and increased T-cell recognition.

Testing for potential off-target reactivity is essential to detect possible safety concerns prior to clinical use. Mapping of TCR fine-specificity using a library of single amino acid substituted peptides followed by a bioinformatic screen did not identify cross-recognized peptides, although this does not completely exclude potential recognition of peptide sequences that are unrelated to the cognate peptide. TCR^{FLT3D/Y} cells did not, however, react to a panel of 26 HLA-A2^{pos} cell lines of different tissue origins, and spared normal blood cells and AML cells expressing the FLT3 WT sequence or alternative D835 mutations.

Treatment with TCR^{FLT3D/Y} cells would be limited to the 3-4% of patients that in addition to expressing the mutation also express HLA-A*02:01 in the Caucasian population. While recurrent FLT3 mutations are frequently known to represent secondary and accelerating AML mutations, our re-analysis of 58 published FLT3 D835Y-mutated AML cases demonstrated that this FLT3 mutation frequently is clonal, and in some cases also might be the initiating mutation or secondary only to a CH mutation. These data are in agreement with the high VAF for FLT3^{D835Y} in all included AML patients harboring this mutation, where the only selection criterion up front was presence of the mutation. Taken together, this highlights the therapeutic and potentially curative potential of eradicating FLT3^{D835Y} mutated AML clones. Furthermore, in cases where TKIs effectively target FLT3 ITD but select for AML subclones with FLT3 point mutations, including FLT3^{D835Y} resistant to 1^{st} as well as 2^{nd} generation TKIs, FLT3^{D835Y} mutation specific TCR treatment could be combined with TKIs. As part of a paradigm where combination regimens are tailored based on tumor molecular profiles, TCR therapies targeting specific recurrent point mutations provide a potential means for highly efficacious eradication of specific tumor clones.

TCRs can access recurrent mutations currently inaccessible to CARs. Moreover, TCRs might have inherent advantages relative to CARs that improve T-cell persistence and antigen sensitivity. Today, TCR-based therapies are mostly applied in the form of genetically modified T cells, although soluble bispecific TCR engagers have emerged as new opportunities for off-the-shelf therapies at lower cost. The results presented here show that a TCR targeting a single shared neoantigen generated from a healthy donor can provide highly efficacious and specific cancer treatment *in vivo* in multiple disease-relevant models, paving the way for future off-the-shelf, tumor-specific immunotherapies.

The proteins disclosed herein are suitable for specific binding to a human leukocyte antigen (HLA) type A2 presenting an HLA-class 1 peptide comprising the sequence YIMSDSNYV (SEQ ID NO: 1).

By "specific binding" is meant that the protein binds specifically to its target under physiological conditions. Accordingly, a protein for specific binding to a human leukocyte antigen type A2 (HLA-A2) presenting an HLA-class 1 peptide will bind to the HLA-A2 if the cognate HLA-class 1 peptide is presented, but is unlikely to bind when a random HLA-class 1 peptide is presented. Accordingly, a protein for specific binding to a HLA-A2 presenting YIMSDSNYV (SEQ ID NO: 1) will bind to the HLA-A2 if YIMSDSNYV (SEQ ID NO: 1) is presented, but display little or no binding to HLA type A2 when for instance DIMSDSNYV (SEQ ID NO: 18) is presented.

HLA-class 1 peptides herein comprise 9 or 10 amino acid residues.

The target antigen in the present disclosure is HLA-A2 presenting an HLA-class 1 peptide comprising or consisting of YIMSDSNYV (SEQ ID NO: 1).

Specific binding to a target may be distinguished from off-target or non-specific binding. For example, the proteins herein bind their targets with a higher affinity than they bind other molecules (or at least most other molecules). The binding of a protein to a target may be measured by any suitable method known in the art, e.g. surface plasmon resonance (SPR) or by binding of soluble, multimerized peptide-HLA complexes to T cells expressing the TCR.

A full-length α-chain comprises, from N-terminal to C-terminal, a variable domain with three CDRs, an extracellular constant domain, a transmembrane domain and a cytoplasmic domain. One example of a full-length α-chain is represented by SEQ ID NO: 12. Another example of a full-length α-chain is represented by SEQ ID NO: 14.

A full-length β-chain comprises, from N-terminal to C-terminal, a variable domain with 3 CDRs, an extracellular constant domain, a transmembrane domain and a cytoplasmic domain. One example of a full-length β-chain is represented by SEQ ID NO 13. Another example of a full-length α-chain is represented by SEQ ID NO: 15.

TCRs herein comprise a full-length α-chain and a full-length β-chain.

An α-chain variable domain comprises three complementarity-determining regions (CDRs); CDR1, CDR2 and CDR3 numbered from the N-terminal. Said CDRs are flanked by framework regions. An α-chain variable domain can thus be represented: N-FRAMEWORK1**CDR1**FRAMEWORK2**CDR2**FRAMEWORK3**CDR3**FRAMEWORK4

In particular, the α-chain CDR1 is represented by TTSDR (SEQ ID NO: 2)

In particular, the α-chain CDR2 is represented by LLSNGAV (SEQ ID NO: 3)

In particular, the α-chain CDR3 is represented by CAVVGGSQGNLIF (SEQ ID NO: 4)

One example of an α-chain variable domain is set forth in SEQ ID NO: 8. Other α-chain variable domains may have more than 90% sequence identity when compared to SEQ ID NO: 8, provided they comprise the same three CDRs. Other α-chain variable domains may have more than 95% sequence identity when compared to SEQ ID NO: 8, provided they comprise the same three CDRs.

A β-chain variable domain comprises three complementarity-determining regions (CDRs); CDR1, CDR2 and CDR3 numbered from the N-terminal. Said CDRs are flanked by framework regions. A β-chain variable domain can thus be represented: N-FRAMEWORK1**CDR1**FRAMEWORK2**CDR2**FRAMEWORK3**CDR3**FRAMEWORK4

In particular, the β-chain CDR1 is represented by MDHEN (SEQ ID NO: 5)

In particular, the β-chain CDR2 is represented by SYDVKM (SEQ ID NO: 6)

In particular, the β-chain CDR3 is represented by CASSLSPGGGYGYTF (SEQ ID NO: 7)

One example of a β-chain variable domain is set forth in SEQ ID NO: 9. Other β-chain variable domains may have more than 90% sequence identity when compared to SEQ ID NO: 9, provided they comprise the same three CDRs. Other β-chain variable domains may have more than 95% sequence identity when compared to SEQ ID NO: 9, provided they comprise the same three CDRs.

An α-chain variable domain can together with a β-chain variable domain form an antigen binding unit. Such antigen binding units may position the six CDRs to specifically bind to a target under physiological conditions.

The minimal antigen binding unit comprises an α-chain variable domain and a β-chain variable domain. The α-chain variable domain and the β-chain variable domain may be connected by a peptide linker from the C-terminal of the first variable domain to the N-terminal of the second variable domain.

The C-terminal of an α-chain variable domain may be fused to the N-terminal of an extracellular α-chain constant domain like in the full-length α-chain. One example of an extracellular α-chain constant domain is set forth by SEQ ID NO: 10.

The C-terminal of a β-chain variable domain may be fused to the N-terminal of an extracellular β-chain constant domain like in the full-length β-chain.One example of an extracellular β-chain constant domain is set forth by SEQ ID NO: 11.

Without being bound by theory, interactions between the α-chain constant domain and the β-chain constant domain may stabilize the antigen binding unit. In one embodiment, the first chain comprises an extracellular α-chain constant domain (located C-terminal to the variable domain, for example directly C-terminal such that the N-terminal amino acid of the constant domain is joined by a peptide bond to the C-terminal amino acid of the variable domain). In another embodiment, the second chain comprises an extracellular β-chain constant domain (located C-terminal to the variable domain, for example directly C-terminal to the variable domain). In an embodiment, both the first chain and the second chain comprise an extracellular constant domain.

Any suitable extracellular constant domains may be used. The constant domains may be human TCR constant domains, or TCR constant domains from another animal, for instance the constant domains may be murine constant domains. Alternatively, the constant domains may be synthetic constant domains, or constant domains derived from a synthetic protein. When the first and second chains both comprise constant domains, both constant domains may be from the same species or different species.

The α-chain variable domain and the β-chain variable domain may be located in two separate chains like in naturally occurring TCRs. Thus, proteins comprising an α-chain variable domain and a β-chain variable domain, which together form an antigen binding unit, may have many different formats. Apart from conventional αβTCRs, single chain TCRs are known and soluble fragments of TCRs are also known.

Accordingly, the polypeptide chain comprising the α-chain variable domain and polypeptide chain comprising the β-chain variable domain may be joined covalently or non-covalently as desired.

In an embodiment, the separate polypeptide chains of the proteins disclosed herein, may be non-covalently joined by dimerization domains like coiled coils with opposite charges. In one embodiment, leucine zippers are used to non-covalently join the chains. Leucine zippers, and their sequences, are well-known in the art, and are reviewed in e.g. Busch & Sassone-Corsi (Trends in Genetics 6: 36-40, 1990). Combinations of covalent and non-covalent interactions may also be used to join truncated or full-length α- and β-chains.

In an embodiment, the separate polypeptide chains of the proteins disclosed herein, may be covalently joined (e.g. by one or more disulphide bonds formed between the side chains of cysteine residues). For instance, it is known that the extracellular constant domains of natural αβTCRs form an interchain cysteine-bridge, which is believed to stabilize the heterodimer.

It is also known that an additional cysteine bridge may be introduced by insertion or by substituting a suitable amino acid residue on each chain with a cysteine residue. The additional cysteine residues should be introduced at locations such that formation of a disulphide bond between the introduced residues leads to the formation of a functional complex in which the variable regions are correctly located and orientated with respect to each other, e.g. as disclosed by Boulter, J.M. et al. (Protein Eng. Des. Sei. 16(9): 707-711, 2003).

Known suitable locations in human TCR chain constant regions for the introduction of cysteine residues are disclosed in Cohen et al. (Cancer Research 67(8):3898-903, 2007) and WO 2019/166463. In the human TCR α-chain constant region of SEQ ID NO: 10, an additional cysteine residue may be introduced by substitution of the threonine residue at position 49 for a cysteine residue. In the human TCR β-chain type 1 constant region of SEQ ID NO: 11, an additional cysteine residue may be introduced by substitution of the serine residue at position 57 for a cysteine residue.

Thus, in one embodiment, the protein disclosed herein comprises an extracellular α-chain constant domain comprising the amino acid sequence set forth in SEQ ID NO: 10, or an amino acid sequence having at least 90 % or 95 % sequence identity thereto; and the second chain comprises an extracellular β-chain constant domain comprising the amino acid sequence set forth in SEQ ID NO: 11, or an amino acid sequence having at least 90 % or 95 % sequence identity thereto. These constant domains may comprise cysteine residues able to form one, two or three interchain cysteine bridges.

Various receptors comprising an α-chain variable domain and a β-chain variable domain are known. Such receptors usually comprise one or two transmembrane domains. An example of such receptor comprising a single transmembrane domain is provided in WO2019069125. An example of such receptors comprising two transmembrane domains are conventional αβTCRs.

The C-terminal of an extracellular α-chain constant domain may be fused to the N-terminal of a transmembrane domain like in the full-length α-chain. One example of a suitable α-chain transmembrane domain is LSVMGLRILLLKVAGFNLLMTLRL, set forth by SEQ ID NO: 21.

The C-terminal of an extracellular β-chain constant domain may be fused to the N-terminal of a transmembrane domain like in the full-length β-chain. One example of a suitable β-chain transmembrane domain is SATILYEILLGKATLYAVLVSGLVLM, set forth by SEQ ID NO: 22.

The C-terminal of a transmembrane domain may be fused to the N-terminal of an intracellular signaling domain like in the full-length α-chain or in the full-length β-chain. An α-chain intracellular domain, WSS, is represented by SEQ ID NO: 23. A β-chain intracellular domain, AMVKKKNS, is represented by SEQ ID NO: 24.

Other intracellular signaling domains may also be used. A variety of intracellular signaling domains are known, and they can be combined and tailored to fit the endogenous signaling machinery in the immune cells. In one embodiment, the intracellular signaling domain comprises a "signal 1" domain like the signaling domains obtainable from the human proteins CD3ζ, FcR-γ, CD3ε etc. In general, it is believed that "signal 1 "-domains (e.g. CD3ζ signaling domain) convey a signal upon antigen binding. In another embodiment, the intracellular signaling domain comprises a costimulatory domain. Such domains are well known and often referred to as "signal 2" domains, and they are believed to, subsequently of "signal 1" domains, convey a signal via costimulatory molecules. Examples of such commonly used human "signal 2" domains include 4- 1BB signaling domain, CD28 signaling domain and ICOS signaling domain.

For efficient expression of the claimed proteins in immune cells, a conventional leader sequence may be introduced N-terminally for facilitating location in the cell membrane. The leader sequence is believed to be trimmed off and will likely not be present in the functional receptors in the cell membrane.

Following synthesis, soluble proteins may be isolated and purified, using techniques known in the art. For instance, the protein may be produced using a host eukaryotic cell. The protein may be expressed such that it comprises a leader sequence which directs it for export. In this case, the protein is exported from the production cell into the culture medium. The culture medium is then separated from the production cell, e.g. by centrifugation.

Soluble proteins provided herein (i.e. proteins lacking transmembrane domains, e.g. the minimal antigen binding unit and soluble TCRs) may be linked or conjugated to a therapeutic or diagnostic agent, or a carrier which comprises or contains a therapeutic or diagnostic agent. A therapeutic agent is an agent used in therapy. By therapy is meant the treatment or prevention of a disease. The therapeutic agent may be an agent useful in the treatment of a neoplastic condition, particularly cancer.

Also provided herein are recombinant nucleic acid molecules encoding the proteins. The nucleic acid molecules provided herein may be an isolated nucleic acid molecule and may include DNA or RNA or chemical derivatives of DNA or RNA. The term "nucleic acid molecule" specifically includes single- and double-stranded forms of DNA and RNA. The nucleic acid (e.g. DNA or RNA) may be circular or linear. A "recombinant" nucleic acid molecule is a nucleic acid molecule synthesized using recombinant techniques, e.g. molecular cloning.

The recombinant nucleic acid molecule provided herein encodes a protein as provided herein. In particular, such recombinant nucleic acids may have promoters allowing expression of the proteins in cellular systems.

The recombinant nucleic acid molecule or construct provided herein may be provided within a vector. The term "vector" as used herein refers to a vehicle into which the nucleic acid molecule or construct provided herein may be introduced (e.g. be covalently inserted) from which the specific binding molecule encoded by the nucleic acid molecule may be expressed and/or the nucleic acid molecule/construct cloned. The vector may accordingly be a cloning vector or an expression vector.

The recombinant nucleic acid molecule provided herein may be provided within a recombinant construct comprising the recombinant nucleic acid molecule linked to a heterologous nucleic acid sequence. By "heterologous" as used herein is meant a nucleic acid sequence which is not natively linked to the nucleic acid molecule described herein, i.e. which is not linked to the nucleic acid molecule described herein in nature. The term "linked" as used herein with respect to the construct may simply mean that the nucleic acid molecule is directly joined to a heterologous nucleic acid sequence. In one embodiment, in the recombinant construct the nucleic acid molecule provided herein is operatively linked to a heterologous expression control sequence.

The term "expression control sequence" refers to nucleotide sequences located upstream of, within, or downstream of a coding sequence, and which influence transcription, RNA processing or stability, or translation of the associated coding sequence (i.e. which influence any aspect of expression of the encoded specific binding molecule). Expression control sequences include promoters, promoter elements such as a TATA box or a B recognition element, operators, enhancers, translation leader sequences, terminator sequences and suchlike.

In one particular embodiment herein, the recombinant nucleic acid molecule comprises a cDNA molecule. By "cDNA" as used herein is meant cDNA in its true and original sense (i.e. DNA synthesized by reverse transcription of mRNA), DNA amplified from original cDNA, and also DNA that is equivalent to cDNA. DNA that is equivalent to cDNA is DNA that encodes a protein as provided herein and that lacks introns, such that it resembles a protein-coding sequence obtained by reverse transcription of mRNA. In one particular embodiment herein, the nucleic acid molecule encoding a protein as provided herein is codon-optimized, in particular the binding protein may be encoded by a codon-optimized cDNA sequence.

The Examples herein demonstrate expression of a TCR from a vector encoding the α-chain and β-chain separated by a porcine teschovirus-derived 2A peptide (also referred to as P2A). A nucleic acid molecule provided herein may thus encode a protein as provided herein in the form of a single polypeptide comprising a first chain (e.g. an α-chain) linked to a second chain (e.g. β-chain) by a selfsplicing 2A peptide. The 2A peptide may have any suitable sequence, such that it has selfsplicing activity. Several 2A peptide sequences are known, any of which may be used according to the present disclosure, e.g. 2A sequences disclosed in Wang et al., 2015 (Scientific Reports 5: Article No. 16273) and WO 2019/166463.

The proteins as provided herein (or, as relevant, the first chain and the second chain of the antigen binding protein) may be encoded with an N-terminal leader sequence. Such leader sequences are believed to direct the proteins to the cell membrane, either for export (in the case of a soluble protein) or insertion into the membrane (in the case of a binding protein comprising a transmembrane domain), and upon export of the protein or its insertion into the membrane, are trimmed off to yield the mature protein.

An exemplary α-chain N-terminal leader sequence may have the amino acid sequence MKSLRVLLVILWLQLSWVWSQ, set forth in SEQ ID NO: 19 and exemplary β-chain N-terminal leader sequences may have the amino acid sequence MGIRLLCRVAFCFLAVGLV, set forth in SEQ ID NO: 20. In the examples below, SEQ ID NO: 19 and SEQ ID NO: 20 are used as leader sequences.

Any suitable leader sequences may be used according to the present disclosure, but in one embodiment the first chain (e.g. α-chain) is encoded with a leader sequence comprising the amino acid sequence set forth in SEQ ID NO: 19, or an amino acid sequence having at least 80 % or 90 % sequence identity thereto; and the second chain (e.g. β-chain) is encoded with a leader sequence comprising the amino acid sequence set forth in SEQ ID NO: 20, or an amino acid sequence having at least 80 % or 90 % sequence identity thereto.

Also provided herein is a kit comprising a first nucleic acid molecule encoding a first chain of a binding protein as provided herein and a second nucleic acid molecule encoding a second chain as provided herein. Thus, the kit comprises a pair of nucleic acid molecules, one encoding a first chain and the other a second chain as described herein.

The two nucleic acid molecules of the kit (e.g. first and second recombinant constructs or first and second vectors) may be provided in a single container (i.e. in a mixture of the two nucleic acid molecules) or in separate containers. The nucleic acid molecules may be provided in an aqueous solution (e.g. in water or a suitable buffer such as TE buffer) or may be provided in a lyophilized form.

As used herein, when referring to "sequence identity" of proteins, an amino acid sequence having at least x% identity to a second amino acid sequence means that x% represents the number of amino acid residues in the first sequence which are identical to their matched amino acid residues of the second sequence when both sequences are optimally aligned via a global alignment, relative to the total length of the second amino acid sequence. Both sequences are optimally aligned when x is maximum by using the comparison matrix BLOSUM62 with gap costs: existence 12, extension 4.

Framework regions may tolerate substitution, addition or deletion of amino acid residues while keeping the ability to position the CDRs for specific binding to a target epitope. As the terminology suggests, the sequences of the constant domains are quite conserved. However, some conservative amino acid substitutions may be tolerated within the constant domains.

The term "conservative amino acid substitution", as used herein, refers to an amino acid substitution in which one amino acid residue is replaced with another amino acid residue having a similar side chain. Amino acids with similar side chains tend to have similar properties, and thus a conservative substitution of an amino acid important for the structure or function of a polypeptide may be expected to affect polypeptide structure/function less than a non-conservative amino acid substitution at the same position. Families of amino acid residues having similar side chains have been defined in the art, including basic side chains (e.g. lysine, arginine, histidine), acidic side chains (e.g. aspartic acid, glutamic acid), uncharged polar side chains (e.g. asparagine, glutamine, serine, threonine, tyrosine), non-polar side chains (e.g. glycine, cysteine, alanine, valine, leucine, isoleucine, proline, phenylalanine, methionine, tryptophan) and aromatic side chains (e.g. tyrosine, phenylalanine, tryptophan, histidine). Thus, a conservative amino acid substitution may be considered to be a substitution in which a particular amino acid residue is substituted for a different amino acid residue in the same family.

The immune cells expressing the disclosed proteins herein may be isolated from a patient or a compatible donor by leukapheresis or other suitable methods. Such primary cells may for example be T cells or NK cells. In particular, autologous T cells (both cytotoxic T cells, T helper cells or mixtures of these) may be transduced with nucleic acids encoding the proteins before a pharmaceutical composition comprising the cells is administered back to the patient. The immune cells expressing the proteins disclosed herein may also be cell lines suitable for clinical use like NK-92 cells. Of course, the preferred cells are human when the intended patient is human.

The pharmaceutical compositions herein can be a composition suitable for administration of therapeutic cells to a patient. The most common administration route for T-cells is intravenous administration. Accordingly, said pharmaceutical compositions may for example be sterile aqueous solutions with a neutral pH. Accordingly, said pharmaceutical compositions may for example be sterile aqueous solutions with a physiological pH. For example, a patient's peripheral blood mononuclear cells may be obtained via a standard leukapheresis procedure. The mononuclear cells may be enriched for T-cells, before transducing them with a lentiviral vector or mRNA encoding the proteins disclosed herein, in particular TCRs. Said cells may then be activated with anti-CD3/CD28 antibody coated beads. The transduced T-cells may be expanded in cell culture, washed, and formulated into a sterile suspension, which can be cryopreserved. If so, the product is thawed prior to administration.

In short, the human β-chain can be represented:
TRBV28-1-CASSLSPGGGYGYTF-TRBJ1 -2

In short, the human α-chain can be represented:
TRAV39-1-CAVVGGSQGNLIF-TRAJ42

### Sequences

SEQ ID NO: 1 Target peptide presented by HLA (9-mer)
   YIMSDSNYV
SEQ ID NO: 2 α-chain CDR1
   TTSDR
SEQ ID NO: 3 α-chain CDR2
   LLSNGAV
SEQ ID NO: 4 α-chain CDR3
   CAVVGGSQGNLIF
SEQ ID NO: 5 β-chain CDR1
   MDHEN
SEQ ID NO: 6 β-chain CDR2
   SYDVKM
SEQ ID NO: 7 β-chain CDR3
   CASSLSPGGGYGYTF
SEQ ID NO: 8 α-chain variable domain 
ELKVEQNPLFLSMQEGKNYTIYCNYSTTSDRLYWYRQDPGKSLESLFVLLSNGAVKQ
EGRLMASLDTKARLSTLHITAAVHDLSATYFCAVVGGSQGNLIFGKGTKLSVKP
SEQ ID NO: 9 β-chain variable domain 
DVKVTQSSRYLVKRTGEKVFLECVQDMDHENMFWYRQDPGLGLRLIYFSYDVKMKEK
GDIPEGYSVSREKKERFSLILESASTNQTSMYLCASSLSPGGGYGYTFGSGTRLTVV
SEQ ID NO: 10 Extracellular α-chain constant domain, human 
NIQNPDPAVYQLRDSKSSDKSVCLFTDFDSQTNVSQSKDSDVYITDKTVLDMRSMDF
KSNSAVAWSNKSDFACANAFNNSIIPEDTFFPSPESSCDVKLVEKSFETDTNLN
SEQ ID NO: 11 Extracellular β-chain constant domain, type 1, human 
EDLNKVFPPEVAVFEPSEAEISHTQKATLVCLATGFFPDHVELSWWVNGKEVHSGVS
TDPQPLKEQPALNDSRYCLSSRLRVSATFWQNPRNHFRCQVQFYGLSENDEWTQDRA
KPVTQIVSAEAWGRADCGFTSVSYQQGVLS
SEQ ID NO: 12 Mature full-length human α-chain without leader sequence 
ELKVEQNPLFLSMQEGKNYTIYCNYSTTSDRLYWYRQDPGKSLESLFVLLSNGAVKQ
EGRLMASLDTKARLSTLHITAAVHDLSATYFCAVVGGSQGNLIFGKGTKLSVKPNIQ
 
NPDPAVYQLRDSKSSDKSVCLFTDFDSQTNVSQSKDSDVYITDKTVLDMRSMDFKSN
SAVAWSNKSDFACANAFNNSIIPEDTFFPSPESSCDVKLVEKSFETDTNLNFQNLSV
IGFRILLLKVAGFNLLMTLRLWSS
SEQ ID NO: 13 Mature full-length human β-chain without leader sequence 
DVKVTQSSRYLVKRTGEKVFLECVQDMDHENMFWYRQDPGLGLRLIYFSYDVKMKEK
GDIPEGYSVSREKKERFSLILESASTNQTSMYLCASSLSPGGGYGYTFGSGTRLTVV
EDLNKVFPPEVAVFEPSEAEISHTQKATLVCLATGFFPDHVELSWWVNGKEVHSGVS
TDPQPLKEQPALNDSRYCLSSRLRVSATFWQNPRNHFRCQVQFYGLSENDEWTQDRA
KPVTQIVSAEAWGRADCGFTSVSYQQGVLSATILYEILLGKATLYAVLVSALVLMAM
VKRKDF
SEQ ID NO: 14 Tested full-length α-chain without leader sequence 
ELKVEQNPLFLSMQEGKNYTIYCNYSTTSDRLYWYRQDPGKSLESLFVLLSNGAVKQEG
RLMASLDTKARLSTLHITAAVHDLSATYFCAVVGGSQGNLIFGKGTKLSVKPDIQNPEP
AVYQLKDPRSQDSTLCLFTDFDSQINVPKTMESGTFITDKCVLDMKAMDSKSNGAIAWS
NQTSFTCQDIFKETNATYPSSDVPCDATLTEKSFETDMNLNFQNLSVMGLRILLLKVAG
FNLLMTLRLWSS
SEQ ID NO: 15 Tested full-length β-chain without leader sequence 
DVKVTQSSRYLVKRTGEKVFLECVQDMDHENMFWYRQDPGLGLRLIYFSYDVKMKEKGD
IPEGYSVSREKKERFSLILESASTNQTSMYLCASSLSPGGGYGYTFGSGTRLTVVEDLR
NVTPPKVSLFEPSKAEIANKQKATLVCLARGFFPDHVELSWWVNGKEVHSGVCTDPQAY
KESNYSYCLSSRLRVSATFWHNPRNHFRCQVQFHGLSEEDKWPEGSPKPVTQNISAEAW
GRADCGITSASYHQGVLSATILYEILLGKATLYAVLVSGLVLMAMVKKKNS
SEQ ID NO: 16 The tested construct with the various domains highlighted 
MGIRLLCRVAFCFLAVGLVDVKVTQSSRYLVKRTGEKVFLECVQDMDHENMFWYRQDPG
LGLRLIYFSYDVKMKEKGDIPEGYSVSREKKERFSLILESASTNQTSMYLCASSLSPGG
GYGYTFGSGTRLTVVEDLRNVTPPKVSLFEPSKAEIANKQKATLVCLARGFFPDHVELS
WWVNGKEVHSGVCTDPQAYKESNYSYCLSSRLRVSATFWHNPRNHFRCQVQFHGLSEED
KWPEGSPKPVTQNISAEAWGRADCGITSASYHQGVLSATILYEILLGKATLYAVLVSGL
VLMAMVKKKNSGSGATNFSLLKQAGDVEENPGPMKSLRVLLVILWLQLSWVWSQELKVE
QNPLFLSMQEGKNYTIYCNYSTTSDRLYWYRQDPGKSLESLFVLLSNGAVKQEGRLMAS
LDTKARLSTLHITAAVHDLSATYFCAVVGGSQGNLIFGKGTKLSVKPDIQNPEPAVYQL
KDPRSQDSTLCLFTDFDSQINVPKTMESGTFITDKCVLDMKAMDSKSNGAIAWSNQTSF
TCQDIFKETNATYPSSDVPCDATLTEKSFETDMNLNFQNLSVMGLRILLLKVAGFNLLM
TLRLWSS
   **Leader sequence**
      β-chain variable domain with three 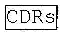
      **Murine extracellular constant domain with Cysteine substitution C**
      **Human transmembrane domain and intracellular domain** *ribosomal skipping sequence P2A*
   **Leader sequence**
      α-chain variable domain with three CDRs 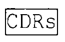
      **Murine extracellular constant domain with Cysteine substitution C**
      **Human transmembrane domain** and **intracellular domain**
   In the examples below, the synthetic TCR^{FLT3D/Y} (SEQ ID NO: 16) was tested. It contained codon-optimized mouse TCRαβ constant domains with additional Cysteine residues to limit mis-pairing, and a porcine teschovirus-derived P2A sequence between the β-chain and the α-chain. The variable domains were also codon optimized to maximize TCR expression and pairing.
SEQ ID NO: 17 Target peptide presented by HLA (10-mer)
   YIMSDSNYVV
SEQ ID NO: 18 Wildtype peptide (9-mer)
   DIMSDSNYV
SEQ ID NO: 19 Leader sequence α-chain
   MKSLRVLLVILWLQLSWVWSQ
SEQ ID NO: 20 Leader sequence β-chain
   MGIRLLCRVAFCFLAVGLV
SEQ ID NO: 21 α-chain transmembrane domain
   LSVMGLRILLLKVAGFNLLMTLRL
SEQ ID NO: 22 β-chain transmembrane domain
   SATILYEILLGKATLYAVLVSGLVLM
SEQ ID NO: 23 α-chain intracellular domain
   WSS
SEQ ID NO: 24 β-chain intracellular domain
   AMVKKKNS
SEQ ID NO: 25 Peptide encoded by minigene
   KICDFGLARYIMSDSNYVVRGNVRLARLP

### Examples

T cells reactive to FLT3^{D835Y}HLA-A*02:01 can be induced by culture of naive healthy donor T cells have not been identified in samples from AML patients at diagnosis or post allo-HSCT.

Naive T cells from a large number of HLA-A2 positive healthy blood donors (n=16) were co-cultured with autologous monocyte-derived dendritic cells (moDCs) electroporated with mRNA encoding a 9-mer and 10-mer peptide with the FLT3^{D835Y} mutation in position 1, predicted to be strong binders to HLA-A*02:01, respectively. The sequence was flanked by 9 - 11 amino acids at the N-terminal and C-terminal end (target mRNA, encoding KICDFGLARYIMSDSNYVVRGNVRLARLP), or control mRNA. After 10 days of co-culture, cells were stained with dual color peptide-MHC (pMHC) multimers complexed with the nonameric or decameric peptide in which the FLT3D/Y mutation was in position one. Multimer^{pos} CD8⁺T cells reactive with the 9-mer were detected in only one donor and only in the culture primed with the target mRNA, and were sorted. Only one functional T-cell receptor sequence (TCR^{FLT3D/Y}) was identified from two clones, and from 55 sequenced sorted single cells. (Fig. 1b).

In contrast, memory cytotoxic T lymphocytes (CTLs) reactive to FLT3^{D835Y}HLA-A2 were not identified among peripheral blood mononuclear cells (PBMCs) from diagnostic AML samples (n=3), or from samples post allo-HSCT with HLA-matched donors (n=3), directly after thawing or following a short 5-day *in vitro* culture in the presence of 100nM of the FLT3D/Y mutant peptide to expand memory T cells.

TCR^{FLT3D/Y} is efficiently expressed in T cells and recognizes a mutant peptide with higher pHLA stability as compared with the corresponding wt peptide

The TCR^{FLT3D/Y} sequence was efficiently expressed in third party peripheral blood (PB) T cells by retroviral transduction, and are called TCR^{FLT3D/Y} cells (Fig. 1c). Binding to HLA-A2, as well as endogenous processing and presentation of the peptide, was demonstrated by immunopeptidomics and targeted mass spectrometry analysis of primary AML cells from two patients, using a monoallelic cell line overexpressing the D835Y mutation as a positive control (Fig. 1d). We previously demonstrated that pHLA stability is strongly predictive of immunogenicity of neoantigens, and the complex of HLA-A2 and mutated peptide had an almost tenfold longer half-life than WT peptide/HLA-A2 (mean 9.9 versus 0.83 hours, *n* = 3) (Fig. 1e). In agreement with this, TCR^{FLT3D/Y} cells stained brightly with pMHC multimers presenting mutant, but not wild type, peptide. A similar fraction of TCR^{FLT3D/Y} re-directed CD8⁺ T cells stained positively with FLT3^{D/Y} pMHC multimers and anti-mouse TCR-β-PE (reactive to the mouse constant region introduced into the TCR and negatively with anti-human TCRα, indicating preferential pairing of the introduced TCR-α and β-chains and suppression of the endogenous TCR. Both CD8⁺ and CD4⁺ T cells exhibited a predominantly naive profile and expanded similarly to T cells transduced with a control TCR *in vitro,* the clinically applied NY-ESO1-specific 1G4 (TCR^{1G4}), indicating lack of fratricide.

TCR^{FLT3D/Y} cells show high peptide sensitivity and specifically recognize the FLT3^{D/Y} epitope in an HLA-A2-restricted manner

The TCR^{FLT3D/Y} cells recognized HLA-A2-transduced K562 target cells pulsed with picomolar concentrations of mutant peptide (EC₅₀=81pM) and displayed no reactivity against the corresponding WT peptide (Fig. 1f). In comparison, TCR^{1G4} cells recognized the cognate NY-ESO1 peptide with an EC₅₀ of 5.5nM, in agreement with previous data showing EC₅₀ of 7.7nM (41). Furthermore, TCR^{FLT3D/Y} cells showed no or negligible reactivity to a panel of 26 HLA-A2^{pos} cell lines of different tissue origins, unless pre-loaded with mutant peptide, suggesting a high degree of peptide and HLA specificity (Fig. 1g).

We next mapped the fine-specificity of TCR^{FLT3D/Y}, TCR^{FLT3D/Y} cells were screened for IFN-γ production in response to target cells loaded with each peptide in a library of 161 peptides representing single amino acid-substituted variants of the cognate peptide (Fig. 1h). Peptide motifs harboring any "permitted" alternative amino acids in each position were queried in the human proteome databases UniProtKB/Swiss Prot by employing the ScanProsite tool (https://prosite.expasy.org/scanprosite/), identifying 28 additional 9mers in the human proteome potentially recognized by the TCR^{FLT3D/Y} cells. However, only three of these peptides (peptide 11 AVISDAMYI, derived from LZTR1; peptide 12 YITSDMFYV, derived from MED1 and peptide 15 AVDNDSFYV, derived from PRADC1) activated TCR^{FLT3D/Y} cells (Fig. li). The three genes encoding proteins harboring the potentially cross-reactive peptide sequences are ubiquitously expressed according to the HPA database (https://www.proteinatlas.org/). Lack of reactivity observed in response to the panel of 26 HLA-A2^{pos} cell lines (Fig. 1g) suggested that the peptides are not processed and presented. To further exclude this, we generated mRNA constructs encoding 30-mer peptides with the cross-reactive peptide in the middle flanked by the natural respective protein sequences, and a GFP reporter. Co-cultures demonstrated that TCR^{FLT3D/Y} cells did not react to K562-A2^{pos} target cells electroporated with the mRNA constructs encoding LZTR1, MED-1 or PRADC1 peptides (Fig. 1j), indicating that these peptides are not naturally processed and presented on HLA-A2. In agreement with this, none of the 28 candidate peptides were found in the HLA-ligand database.

TCR^{FLT3D/Y} cells specifically recognize and kill primary FLT3^{D835Y} AML cells *in vitro*

Re-analysis of publicly available DNA sequencing data of 49 AML cases with the FLT3^{D835Y} mutation demonstrated that the FLT3^{D835Y} mutated clone is frequently dominant. The FLT3^{D835Y} mutation had the highest variant allele frequency (VAF) among identified recurrent driver single nucleotide variants (SNVs)/indels in 22 cases (45%). In two additional cases, it was only preceded by a recurrent *SRSF2* and *TET2* mutation, known to occur in normal individuals with clonal hematopoiesis (CH) and requiring additional driver mutations to transform to AML. Similarly, re-analysis of single-cell DNA sequenced AMLs showed in 2 of 9 cases with the FLT3^{D835Y} mutation that it had the highest VAF or was secondary only to CH associated mutations. Taken together, this analysis of 58 FLT3^{D835Y} mutated AML cases indicates that FLT3^{D835} might in some cases constitute an AML initiating or transforming mutation, and regardless that it frequently is clonal.

We next explored the specificity and efficacy with which TCR^{FLT3D/Y} cells killed AML cells from 11 patients. DNA sequencing of mononuclear samples dominated by myeloid cells (Fig. 2a), showed high FLT3^{D835Y} clonal involvement in the eight FLT3 D835Y^{pos} patients (Fig. 2b). TCR^{FLT3D/Y} cells killed myeloid cells from AML patients 1-7 effectively at effector:target (E:T) ratios as low as 1:2 (mean 87%, range 53.3% - 98.7%, Fig. 2c and 2d). CD3⁺ T cells and CD19⁺CD20⁺ B cells were not significantly affected (Fig. 2c and 2d), as expected in view of low clonal involvement. However, B cell counts were very low, and we therefore also investigated the effect of TCR^{FLT3D/Y} cells on isolated B cells from healthy donors, which demonstrated lack of killing. The HLA-restriction of TCR^{FLT3D/Y} was confirmed by lack of killing of myeloid HLA-A2^{neg} FLT3^{D835Y} patient cells (pt. 8, Fig. 2c and 2d). Specificity for the D835Y substitution was demonstrated by lack of recognition of AML samples with alternative amino acid substitutions in D835 position (pt. 9-10) or expressing FLT3^{WT} (pt. 11) (Fig. 2d). Robust and specific IFN-γ production was observed upon co-incubation of TCR^{FLT3D/Y} cells with HLA-A2^{pos} FLT3^{D835Y} patient cells. Finally, TCR^{FLT3D/Y} cells derived from AML patients killed autologous leukemia cells with similar efficacy and selectivity as third party T cells, mimicking a clinical setting (Fig. 2e). In most cases, 2-4 experiments were performed per patient sample, with only 1 experiment performed due to limited amount of material in a few instances, as described in figure legends.

TCR^{FLT3D/Y} cells efficiently target human leukemia cells *in vivo*

We initially investigated if TCR^{FLT3D/Y} cells recognized endogenously presented antigen in a small experiment using an *in vivo* xenograft mouse model engrafted with a leukemia cell line. Since FLT3^{D835Y} leukemic cell lines are not commercially available, we introduced the mutation into different leukemia cell lines and demonstrated that TCR^{FLT3D/Y} cells killed >95% of the leukemic cells in 24h at low E:T ratios (1:2). BV173^{D835Y} cells (cell line origin: B cell precursor leukemia) were next transplanted into NOD-scid IL2Rg^{null} (NSG) mice. All mice in the control groups (untreated (n=3) and TCR^{1G4} cells (n=3)) were sacrificed after 21 days due to high leukemic burden, at which time leukemic cells were undetectable in the TCR^{FLT3D/Y} cell-treated group (n=4). Among four mice receiving the therapeutic TCR, two survived for the duration of the experiment (52 days), one remained leukemia free but was sacrificed on day 41 due to GVHD, whereas one relapsed day 28 day and was found dead on day 32 (see Figure 6). Upon termination, no leukemic cells were detected in the bone marrow (BM) of the two surviving TCR^{FLT3D/Y} cell-treated mice, while transduced T cells in PB and BM persisted.

TCR^{FLT3D/Y} cells efficiently eliminate primary AML *in vivo* in patient-derived xenograft models, including AML-propagating cells

To investigate the *in vivo* efficacy of TCR^{FLT3D/Y} cells in disease-relevant models, we next treated mice engrafted with primary AML cells in different patient-derived xenograft models.

### Model 1

NSG-SGM3 mice highly engrafted with primary FLT3^{D/Y} AML cells from patient 7 (24.5 ± 2.2% human CD33⁺ cells in PB) (Fig. 3a-b) were treated with TCR^{FLT3D/Y} (*n* = 7) or control TCR^{1G4} *(n* = 6) cells. Serial analysis of PB demonstrated a maintained high CD33⁺ engraftment in TCR^{1G4} -treated mice, whereas CD33⁺ cells were virtually eliminated by day 14 in all TCR^{FLF3D/Y}-treated mice (Fig. 3b). Mice were closely monitored for potential allo-reactivity mediated by endogenous TCRs of the TCR-transduced T cells derived from a third-party donor, which could otherwise confound the anti-tumor reactivity mediated by the TCR^{FLT3D/Y}. Thus, terminal analysis of all TCR^{1G4}-treated and TCR^{FLT3D/Y}-treated mice was performed on day 15 after T-cell infusion when tumor burden started to decline slightly also in control mice (Fig. 3b). BM analysis demonstrated high CD33⁺ AML engraftment in TCR^{1G4}-treated mice (mean 86.9 ± 5.3%), which was reduced to mean 0.5 ± 0.2% in TCR^{FLT3D/Y}-treated mice, with a similar reduction in the spleen (Fig. 3c-d). An almost complete elimination of FLT3^{D/Y} AML cells in BM was confirmed by ddPCR analysis, quantifying clonally involved cells (Fig. 3e). Importantly, efficient targeting of human AML cells in TCR^{FLT3D/Y-}treated mice resulted in recovery of mouse hematopoiesis, which was severely suppressed by high leukemic burden in TCR^{1G4}-treated mice (Fig. 3f). TCR-transduced T cells were detected in BM, spleen and PB of all mice throughout the experiment (Fig. 3g).

### Model 2

Primary xenografts from a second FLT3^{D/Y} mutated AML engrafted in NSG-SGM3 mice (patient 1) were investigated for response to TCR^{FLT3D/Y} cells (Fig. 4a). Unlike patient 7 (Fig. 3c), this AML co-expressed CD34, a stem/progenitor marker characteristic of CD34⁺ AMLs (46), for which CD34⁺ in contrast to CD34⁻ cells have been shown to possess leukemia propagating activity (47, 48). Control TCR-treated mice had relatively low levels of PB human CD33⁺ engraftment 34 days after T-cell injection (mean 6.0 ± 3.1% hCD45⁺CD33⁺ cells), whereas BM engraftment was much higher (mean 23.7 ± 5.8%) (Fig. 4b). In TCR^{FLT3D/Y} treated mice this was reduced to 1.9 ± 0.7% in PB and 1.7 ± 0.3 % in BM. Terminal BM analysis day 34 after T-cell injection revealed distinct populations of CD33⁺CD34⁻(mean 19.4 ± 4.8%) and CD33⁺CD34⁺ cells (mean 4.4 ± 1.0 %) in control TCR^{1G4}-treated mice (Fig. 4b and 4c). In TCR^{FLT3D/Y} treated mice some CD33⁺CD34⁻ cells persisted in BM although at greatly reduced levels (mean 1.7 ± 0.3%), whereas CD33⁺CD34⁺ cells were completely eliminated. Similar findings were observed in PB and spleen (Fig. 4c).

DNA sequencing confirmed that cells engrafted in NSG-SGM3 mice were represented by the same exonic mutations as those detected in AML blasts from BM of patient 1, with FLT3^{D835Y} and WT1^{H507P} representing the only detected recurrent driver mutations. Further quantification by ddPCR demonstrated that FLT3^{D835Y} and WT1^{H507P} mutations represented the dominating clone, with a VAF >45% in patient 1 as well as in engrafted NSG-SGM3 mice (Fig. 4d). Notably, among the remaining CD33⁺CD34⁻ BM cells of TCR^{FLT3D/Y} treated mice, the fraction of FLT3^{D/Y} mutated cells was reduced to only 0.9 ± 0.3% as compared with 83.4 ± 2.1% in TCR^{1G4}-treated mice. This translates into more than a 1300-fold reduction in CD33⁺CD34⁻FLT3^{D/Y} AML cells in TCR^{FLT3D/Y} treated mice (Fig. 4d-e). Human T cells including TCR-transduced T cells were detected in BM, spleen and PB of all treated mice throughout the course of the experiment (Fig. 4f). Cells from patient 1 also reconstituted CD19⁺ B lymphocytes in engrafted mice (Fig. 4b), and in agreement with previous studies, these were not part of the FLT3^{D/Y} leukemic clone.

### Model 3

To establish a PDX model mimicking minimal residual disease (MRD), secondary transplantations of mice engrafted with patient 1 AML cells were performed into NSG mice, a mouse strain that in contrast to NSG-SGM3 mice does not specifically enhance human myeloid lineages. In accordance with the results from the NSG-SGM3 model, we observed efficient elimination of the FLT3^{D/Y} primary AML cells also in this MRD setting (Fig. 5a-b).

### Model 4

TCR^{FLT3D/Y} treatment resulted in undetectable levels of CD34⁺ FLT3^{D/Y} AML cells in PB, spleen and BM of all mice engrafted with AML cells from patient 1, compatible with complete elimination of FLT3^{D/Y} AML propagating cells *in vivo* through specific TCR^{FLT3D/Y} targeting. More definitive support for this would, however, require longer follow up of mice to allow for potential outgrowth of rare and therapy-resistant AML stem cells that might have escaped T-cell recognition. A long follow-up time was not possible in models 1-3 due to continued presence of TCR T cells having endogenous TCR repertoires that cause allo-reactivity and xenoreactivity over time. Moreover, to establish whether *all* AML-propagating cells have been eliminated by the TCR^{FLT3D/Y}, requires assessment in the absence of TCR^{FLT3D/Y} T cells. To circumvent these limitations patient 1 AML cells were cultured with TCR^{FLT3D/Y} or TCR^{1G4} cells or without T cells *in vitro* for 48 hours prior to transplantation into NSG mice (Fig. 5c). The very few human T cells to which the AML cells were exposed *in vitro* did not persist *in vivo.* The mice did not get infusions of IL-2. Potential AML development could therefore be followed for 28 weeks. During this time, mice injected with AML cells from control cultures without T cells or with control TCR^{1G4} cells showed progressive and high leukemic engraftment. In contrast, no detectable engraftment was observed at any time in any mice transplanted with AML cells co-cultured with TCR^{FLT3D/Y} cells (Fig. 5d). Collectively, these experiments demonstrate that TCR^{FLT3D/Y} cells can efficiently target and eliminate FLT3^{D/Y} mutated *in vivo* AML propagating cells.

### Methods

Primary patient cells, healthy blood donor cells and cell lines
PBMCs from healthy donor buffy coats were obtained from the blood bank of Oslo University Hospital, and PB or BM mononuclear cells from leukemia patients were from biobanked, cryopreserved material (ethical approval # 2018/879 and 2018/1246). Mononuclear cells were isolated by density-gradient centrifugation (Axis-Shield) and were typed for HLA-A2 expression by flow cytometry. To confirm the presence of FLT3^{D835Y}, genomic DNA was extracted (QIAGEN DNeasy purification kit) from patient primary cells, and samples were sequenced using the TruSight^{™} Myeloid Panel (Illumina, San Diego, CA, USA).

Epstein Barr-virus transformed lymphoblastoid cell lines (EBV-LCL) were generated from HLA-A2^{pos} and HLA-A2^{neg} PBMCs as described previously (68). The following cell lines were obtained from American Type Culture Collection (ATCC) or German Collection of Microorganisms and Cell Cultures (DSMZ): NALM-6, BV173, RS4;11, T2, RD, U-2 OS, FM-6, UT-7, HeLa, HaCaT, MCF-7, K562, ML-2, COLO 668, EA.hy926, U-87 MG, Daoy, HCT-116, CHP-212, OCI-M2, HEK 293, Hep G2, MV-4-11, EoL-1, MOLM-13, Caco-2, HEL 92.1.7, B721.221, Phoenix-AMPHO. All cell lines were cryopreserved in aliquots labeled according to passage and only low passage cell lines were used to start fresh cultures. The identity of cell lines was ascertained by short tandem repeat DNA profiling, a service provided by Labcorp DNA Identification Lab, NC, USA (formerly Genetica, https://celllineauthentication.com/). Cell lines were cultured in humidified cell-incubators containing 5% CO₂ at 37 °C in the media instructed by the supplier, and regularly tested for mycoplasma contamination.

### Minigene Design

For generation of T-cell responses a minigene was designed to encode predicted epitopes (https://services.healthtech.dtu.dk/service.php?NetMHC-4.0) containing the FLT3^{D835Y} mutation, codon-optimized and synthesized by Genscript. Subsequently, it was cloned into the pCIpA102 vector for *in vitro* mRNA transcription using the RiboMAX LargeScale RNA production system (Promega), as previously described (69, 70). The minigene encoded the FLT3 amino acid sequence KICDFGLARYIMSDSNYVVRGNVRLARLP.

### Induction of antigen-specific T cells

Monocytes from PBMCs of HLA-A2^{pos} healthy donors were isolated on day -4 using CD14-reactive microbeads and AutoMACS Pro Separator (Miltenyi Biotec), while the remaining CD14^{neg} PBMC fraction was cryopreserved for later use. The monocytes were then cultured for three days in CellGro GMP DC medium (CellGenix) supplemented with 1% (vol/vol) human serum (HS, Trina biotech), 1% (vol/vol) penicillin/streptomycin (P/S, Sigma-Aldrich), 50 IU/ml Interleukin (IL)-4 (PeproTech) and 800 IU/ml GM-CSF (Genzyme). Subsequently, the monocyte-derived-dendritic cells (MoDC) were matured for 14-16 h by adding lipopolysaccharide (LPS; Sigma-Aldrich) and IFN-γ (PeproTech) to final concentrations of 10 ng/ml and 100 IU/ml respectively. On day -1, naive CD8⁺ T cells were isolated from the autologous CD14^{neg} cryopreserved PBMCs by use of AutoMACS Pro Separator and CD8⁺ T-cell isolation kit mixed with CD45RO- and CD57-reactive beads (Miltenyi Biotec). On day 0, MoDCs were harvested, electroporated with mRNA and co-cultured with naive T cells in DC-T cell medium supplemented with 30 ng/ml IL-21 (PeproTech) at a DC:T cell ratio of 1:4. After 10 days, co-cultures were screened for the presence of FLT3^{D/Y} pMHC multimer-reactive CD8⁺ T cells. pMHC multimers labeled with (PE) and (APC) were prepared in-house as described previously (71, 72). Live CD8⁺ T cells staining positively for PE- and APC-conjugated pMHC multimers were sorted subsequently by fluorescence activated cell sorting (FACS).

Expansion of potential memory T cells reactive to FLT3^{D/Y} in samples from AML patients following HSCT

For *in vitro* expansion of potential memory T cells reactive to the FLT3^{D/Y} mutant peptide in AML patients that had undergone HSCT, cryopreserved PB samples were thawed and resuspended in Iscove's Modified Dulbecco's (IMDM) supplemented with 20% (vol/vol) FCS (Trina biotech) and 0.1mg/ml DNase. Between 2-4 mill viable cells were recovered and were resuspended at a concentration of 1M/ml and pulsed with the FLT3^{D/Y} peptide at 100ng/ml for 2 hours at 37°C. Cells were then washed and resuspended at a concentration of 3.75M/ml in IMDM 5% HS, 1x P/S and 20 U/mL IL-2 before culture for 5 days. Identification of T cells reactive to the peptide was performed by staining with pMHC multimers conjugated to PE and APC as described above.

Sorting and cloning of pMHC multimer^{pos} CD8⁺ T cells PBMCs from three healthy donors were isolated, mixed in equal ratio (1:1:1) and were then irradiated with 35 Gy, washed and resuspended in X-vivo 20 medium (Lonza, BioNordika) supplemented with 5% HS and 1% P/S (T-cell medium). 0.2 × 10⁶ irradiated cells (feeders) were placed into tissue culture treated 96-well plates and were supplemented with 100 µL of T-cell medium containing 2 µg/ml phytohaemagglutinin (PHA; Remel Thermo Scientific), 80 ng/ml IL-2 (R&D Systems) and 4 ng/ml IL-15 (PeproTech). The FLT3^{D/Y} co-cultures were then harvested and stained with LiveDead Fixable Near-IR, anti-CD3, -CD8, PE- and APC-conjugated pMHC multimers and using the FACS Aria II (BD Biosciences) cell sorter, CD8⁺, pMHC double positive multimer populations were sorted as single cells into 96-well plates containing feeders. After 7 days, cultures were supplied with fresh T-cell medium containing 1750 U/ml IL-2 and 4ng/ml IL-15, and expanding clones were identified by microscopy. At day 14 growing clones were restimulated with feeder cells prepared as described above and stained with FLT3^{D/Y} pMHC multimers. To assess functionality after further expansion, the clones were stimulated with K562 cells pulsed with FLT3^{D/Y} or FLT3^{WT} peptides and assessed for upregulation of CD137.

### TCR sequencing

Paired TCRα and β chains from two clones and 55 single cells reactive to FLT3^{D/Y} were amplified using a protocol described previously that was modified and adapted for the targeted amplification of TCRα and β transcripts (37, 54, 68). MiXCR script was used to analyze sequencing data, and an in-house python script TCR primer was used to reconstruct full-length TCR chains as described previously (54, 73). Output was manually verified for each sample in IMGT/V-Quest (74). Variable TCR-α and TCR-β fragments of identified TCRs were codon-optimized, synthesized and cloned by Genscript.

### Gene transfer to human PBMCs and cell lines

The FLT3^{D/Y} and NY-ESO-1 (1G4) specific TCRs were transduced into HLA-A2^{pos} healthy donor-derived and patient-derived PBMCs for both *in vitro* and *in vivo* experiments. For stimulation of human PBMCs, 12-well tissue culture-treated plates were coated with anti-CD3 (clone OKT3, eBioscience) and anti-CD28 (clone CD28.6, eBioscience) antibodies for at least 2 hours at 37°C. 2 × 10⁶ cells/ml PBMCs in CellGro GMP DC medium supplemented with 5% (vol/vol) human serum (HS, Trina biotech), IL-7 and IL-15 (5 ng/ml each, PeproTech) were added to antibody-coated plates and incubated at 37°C and 5% CO₂ for 72 h. For generation of retroviral supernatants, 4 × 10⁶ Phoenix-AMPHO packaging cells were plated in 10 cm petri dishes for 24 h and cells were transfected with γ-retroviral vector DNA, mixed in X-tremeGENE 9 DNA Transfection reagent (Roche Diagnostics) and Opti-MEM. Next day, medium was refreshed, and cells were incubated at 32°C and 5% CO₂ for 24 h. Subsequently, PBMCs were harvested, resuspended in CellGro GMP DC medium supplemented with 5% human serum, IL-7 and IL-15, mixed with retroviral supernatant, placed in non-tissue culture treated 6-well plates pre-coated with Retronectin (20 µg/ml, Takara) and spinoculated at 900 g for 60 min. A second spinoculation was performed the following day with fresh retroviral supernatant and transduction efficiency was determined after 3 days by staining with anti-mouse TCR-β chain antibody and/or pMHC multimer followed by flow cytometry. Prior to functional experiments, cells were cultured for 48-72 h in CellGro GMP DC medium containing low concentrations of cytokines (0.5 ng/ml IL-7 and IL-15). Alternatively, cells were frozen for later experiments.

BV173, ML-2, RS4;11 and NALM-6 cell lines were also transduced as described above using retroviral supernatant containing the FLT3^{D/Y} minigene. For *in vivo* experiments, the BV173 cell line was stably transduced to express the FLT3^{D/Y} minigene, firefly luciferase and green fluorescent protein (GFP) and was designated as BV173^{D835Y}. All transduced cell lines were subsequently purified by FACS sorting, expanded and frozen for use in later experiments.

Complementary DNA for FLT3^{D/Y} and HLA-A2 were cloned into the pCIpA102 vector for mRNA production, as previously described (70, 71).

### Immunoprecipitation-Targeted Mass spectrometry (IP-MS) analysis of FLT3 peptides presented on HLA

Mono-allelic B721.221 cells expressing HLA-A*02:01 were transduced using retroviral supernatant encoding the mutant FLT3 amino acid sequence VLVTHGKVVKICDFGLARYIMSDSNYVVRGNARLPVK. One hundred million cells from the B721.221 and four hundred million cells from the patient samples (patient 1 and 3) were each lysed in PBS containing 1% lauryl maltoside, 0.5mM EDTA, 1mM PMSF and Sigma protease inhibitors (1:200) for 1h at 4°C (1 mL lysis buffer per 100 million cells). The clarified cell lysates (12.000 RPM, 15 min), were then added to 200uL of AminoLink plus bead slurry (ThermoFisher Scientific) coated with pan HLA class I specific antibody (W6/32, BioXcell) and incubated for 3h at 4°C. The HLA class I antibody was coupled to the AminoLink plus bead slurry, following the manufacturers suggestions (10 mg of W6/32 antibody was coupled to 2 mL of amino link plus bead slurry). Following the 3h incubation with cell lysates, the beads were washed with 3 mL of 0.1M Tris-HCl/150mM NaCl, 3 mL of 0.1M Tris-HCl/400mM NaCl, and again with 3 mL of 0.1M Tris-HCl/150mM NaCl and finally with 3 mL of 0.1 M Tris-HCl. The HLA bound peptides were then eluted with 1 mL of 1% TFA, followed by another two elutions, each with 1mL of 1% TFA. The peptide elutions were pooled and loaded onto the Discovery DSC-C18 SPE column for desalting before MS analysis. The peptides were vacuum concentrated and dissolved in 25 µL of 3% acetonitrile containing 0.1% TFA, following spike-in with 200 picograms of heavy isotope labelled peptide (YI(¹³C₆,¹⁵N)MSDSNYV) prior to targeted MS analysis. The peptide solution (5 µL) was analyzed using an Easy nLC 1000 system (Thermo Fisher Scientific, Sunnyvale, CA, USA) connected to a Q Exactive HF mass spectrometer (ThermoElectron, Bremen, Germany) equipped with a nano electrospray ion source. For liquid chromatography separation, an EASY-Spray ES902 column (C18, 2 µm beads, 100 Å, 75 µm inner diameter) capillary of 25 cm bed length was used. A flow rate of 300nL/min was employed with a solvent gradient of 7-35% B in 55 min, to 90% B in 3 min. Solvent A was 0.1% formic acid and solvent B was 0.1% formic acid/90% acetonitrile. The mass spectrometer was operated in Parallel Reaction Monitoring (PRM) mode, to specifically target the presence of endogenous FLT3 mutant (m/z=1091.4738¹⁺) and spiked-in isotope labelled peptide (m/z=1098.4928¹⁺), eluting within a retention time window of 27-31 minutes, as determined using synthetic analog. The MS/MS spectra using higher-energy collision induced dissociation (HCD) were acquired with the resolution R = 15,000, after accumulation to a target of 1e5. The normalized collision energy was set to NCE 27, isolation window was m/z = 2.0. The maximum allowed ion accumulation for the MS/MS spectrum was 120ms. Raw data were analyzed using the XcaliburTM software and Skyline (MacCoss Lab Software).

### pMHC stability assay

HLA-A*02:01 molecules were prepared in-house, according to previously described approaches (71, 75, 76). The pMHC stability assay was performed as previously described (37), with minor modifications. Briefly, UV-mediated peptide-exchange reactions were performed for 1h, followed by incubation of the resulting product at 4°C. The next day, streptavidin coated beads were washed twice with PBS-Tween 1%. The peptide-HLA monomers were coupled with the washed beads for 10 minutes at room temperature. After coupling, the beads were washed twice with PBS-Tween 1% and resuspended in 200ul. An aliquot of 20ul beads was taken aside for the 0-hour time point, while the remaining beads were incubated at 37°C.

Twenty ul of beads were collected at 3, 6, 12 and 24h of incubation. After each time point collection, the beads were stained with 30 ul of anti-HLA-A2 PE antibody (#343305 Biolegend, 1:100 dilution) for 10 minutes at room temperature. All time points were analyzed immediately after staining on a BD LSR II Flow cytometer. Antibodies, dyes and flow cytometry

For surface antibody staining of human PB and BM cells, antibodies were added to cells for 15-20 min at 4°C followed by washing steps. For intracellular staining, cells were suspended in Cytofix/Cytoperm (BD Bioscience) solution for 20 minutes, washed with Perm/Wash buffer (BD Bioscience) and then stained with antibodies. For mouse PB, BM and spleen, cells were processed into single cell suspension as previously described (77). All samples were Fc-Receptor blocked (human; Miltenyi Biotec, mouse; produced by mouse hybridoma cell line clone 2.4 G2, ATCC, HB-197) for minimum of 10 minutes at 4°C prior to staining with prediluted conjugated antibodies for 15-20 minutes at 4°C. The following fluorescently conjugated anti-human and anti-mouse antibodies were acquired from BD Biosciences or BioLegend unless otherwise specified: Anti-human CD3 (SK7, UCH-T1, HIT3α), - CD4 (OKT4, RPA-T4, SK3), -CD8a (HIT8a, RPA-T8), -CD11b (ICRF44), -CD13 (WM15), -CD14 (HCD14, M5E2, MϕP9), -CD16 (3G8, NKP15), -CD19 (HIB19, SJ25c1, 4G7), -CD20 (2H7), -CD33 (P67.6, VM-53), -CD34 (561, 581, 8G12), - CD38 (HB-7), -CD40 (LOB7/6 Serotec) -CD45 (30-F11, H130), -CD45RA (HI100), -CD45RO (UCHL1), -CD56 (NCAM, N901 Beckman Coulter), -CD57 (QA17A04), -CD62L (DREG-56), -CD123 (6H6), -CD135 (BV10A4H2), -CD137 (4B4-1), - CD197 (G043H7), -HLA-A2 (BB7.1, BB7.2), -HLA-DR (L243), anti-mouse CD45 (30-F11) and - Ter199 (TER119). Anti-mouse TCR-β chain (H57-597) was used to determine transduction efficiency of the TCR^{1G4} or TCR^{FLT3D/Y} in human cells and monitor transduced T cells used for *in vivo* treatment in mice. Live/Dead Fixable Near-IR and Aqua Dead Cell Stain kit (Life Technologies), DAPI (Invitrogen) or 7-AAD (BioLegend) was used to exclude dead cells during flow cytometry. Cell trace violet (CTV) (Life Technologies) was used to label the TCR-transduced PBMCs in T cell activation and killing assays. Human mature blood lineages were gated as single, viable, mTer119 (erythrocyte) negative and further defined as followed: myeloid; hCD45⁺CD33⁺, B cells; hCD45⁺CD19⁺, T cells; hCD45⁺CD19-CD33-CD3⁺CD4⁺/CD8⁺, therapeutic T cells; hCD45⁺CD19-CD33-CD3⁺CD8⁺mTCR-β⁺. The percentage of myeloid was determined as a fraction of combined mouse and human leukocytes (mCD45⁺hCD45⁺), subtracting hCD3⁺ events accounting for infused T cells. FACS analysis was performed on BD LSR II flow cytometer (BD Biosciences) or BD LSRFortessa (BD Biosciences), while cell sorting was performed on FACSAria Fusion (BD Biosciences). Data were analyzed using FlowJo (TreeStar) or FACS DIVA (BD Biosciences) software. To visually display flow cytometry data, we utilized unsupervised nonlinear dimensionality reduction algorithm such as T-Distributed Stochastic Neighbor Embedding (*t*-SNE) by using FlowJo (TreeStar) software.

### T cell activation assays

TCR-transduced T cells were co-cultured with cell lines or primary patient tumor cells at an effector:target cell ratio of 1:2 (100,000:200,000 cells/well) and reactivity was investigated by measuring CD137 upregulation or release of IFN-γ. Where indicated, target cells were pulsed with FLT3^{D/Y} or FLT3^{WT} peptide (purities >90%), or 161 single amino acid substituted variants of the FLT3^{D/Y} peptide (purities >70%) (GenScript Biotech) for 1-2 h or electroporated with mRNA encoding either WT FLT3, or the FLT3^{D/Y} minigene. Cells were placed in round or flat bottom 96-well plates and after 18-20 h of co-incubation centrifuged at 700 g for 2 min. Supernatants were harvested for measurement of IFN-γ by ELISA, while cells were washed and stained with anti-CD137. In some experiments, transduced T cells were labeled with 0.75 µM CTV to distinguish them from target cells. Reagents for IFN-γ ELISA were acquired from BD Pharmingen or R&D Systems: mouse anti-human IFN-γ capture antibody (NIB42), Biotin Mouse Anti-Human IFN-γ detection antibody (4S.B3), streptavidin-HRP, stabilized tetramethylbenzidine and hydrogen peroxide as substrate solutions, sulfuric acid as stop solution, and recombinant human IFN-γ protein as standard. The assay was performed according to manufacturer's instructions.

Flow cytometry-based cytotoxicity assay using cell lines as targets Transduced cell lines stably expressing the FLT3^{D/Y} minigene were co-cultured with CTV-labeled TCR-transduced T cells at an effector:target cell ratio of 1:2 (75,000:150,000 cells/well) for 48 h in round bottom 96-well plates in triplicates. Following co-culture, cells were harvested, washed, and stained with human anti-CD3, -CD8, -CD4 and Live/Dead NIR for 15-20 minutes. Cells were then washed and resuspended in 200 µL of FACS buffer containing 10,000 CountBright Absolute Counting Beads (Thermo Fisher). An equal number of bead events (3,500) were recorded during acquisition of data from every well. Data were normalized and reported as percentage of the mean of live tumor cell numbers acquired from three parallel wells co-cultured with TCR^{1G4} or TCR^{FLT3D/Y} cells from the same donor.

Flow cytometry-based assays for T-cell activation and cytotoxicity using primary human samples
PB or BM samples from AML patients were thawed and resuspended in Iscove's Modified Dulbecco's Medium (IMDM) supplemented with 20% (vol/vol) FCS (Trina biotech) and 0.1mg/ml DNase. Patient cells were centrifuged at 200g for 15 min at room temperature and were transferred to round bottom 96-well plates for assays measuring CD137 upregulation on TCR-transduced T cells or cytotoxicity on target cells. Normal CD19⁺ B cells were isolated from healthy donor buffy coat (BC) mononuclear cells using CD19-reactive microbeads and AutoMACS Pro Separator (Miltenyi Biotec) and were transferred to round bottom 96-well plates for assays measuring cytotoxicity on target cells. Individualized antibody panels and gating strategies to identify malignant blasts and normal leukocyte populations were designed after reviewing diagnostic phenotyping available in the hospital records. Allogeneic or, for patients 1, 2 and 3, also autologous patient-derived T cells transduced with TCRs, were used in the experiments. TCR-transduced cells were pre-labeled with CTV dye to distinguish them from target cells. For cytotoxicity assays, 75,000 T cells per well were co-incubated with 150,000 target cells in 3 parallels per condition for 72 h and then stained with individualized antibody panels for flow cytometry. CountBright Absolute Counting Beads were used as described above.For patients 1-6 and 8, myeloid cells were identified as CD3⁻CD19⁻CD20⁻, normal T cells as CD3⁺ and normal B cells as CD19⁺CD20⁺. Cells from patient 7 were obtained from Jackson Laboratory (Stock ID J000106565) and patient-specific phenotypic markers CD33 and CD19 were used to identify the leukemia cells based on characterization profile from the provider.

*In vivo* FLT3^{D/Y} TCR T cell activity in xenograft leukemia cell line model This study was approved by the Norwegian Food Safety Authority (application ID: 17500). All experiments were performed in compliance with the institutional guidelines and 2010/63/EU directive on the protection of animals used for scientific purposes. 8-10-weeks-old female NOD-*scid* IL2Rg^{null} (NSG) mice bred in-house were used in these experiments. On day -15, mice were sub-lethally irradiated with 2.5 Gy radiation using MultiRad225 X-ray irradiator (RPS services). 4 × 10⁶ or 1 × 10⁶ cells of the human B-ALL cell line BV173^{D835Y} retrovirally transduced to express the FLT3 D835Y mutation and GFP and firefly luciferase were injected on day -14 through the tail vein. After leukemia was established and confirmed by BLI on day -1, mice were treated with 10⁷ T cells, transduced with either TCR^{1G4} or TCR^{FLT3D/Y} . A separate group of control mice did not receive any T cell injections. All mice were injected daily with 2500 IU IL-2 (R&D Systems) by intra-peritoneal (IP) injections. BLI imaging (by IVIS Spectrum *in vivo* imaging system, and analysis by Living image software version 4.5.2, PerkinElmer) and blood analysis by flow cytometry was performed at different intervals. For survival analysis, mice were observed for clinical signs of GVHD, and were sacrificed if they developed more than 20% weight loss, hunched posture, ruffled fur or limb paralysis. The experiment was terminated 7.5 weeks after T-cell injection and surviving mice in treated groups were euthanized by cervical dislocation. In all three experimental cohorts, bone marrows were collected at time of sacrifice and processed for flow cytometry to analyze the presence of T cells and tumor cells.

*In vivo* activity of TCR^{FLT3D/Y} cells in four primary AML patient-derived xenograft models

Experiments were performed according to the guidelines and obtained permissions from Stockholms Djurförsöksetiska nämnd (17978-2018). Experimental mice were housed 2-5 mice per cage in IVC-Mouse GM 500 cages with a light cycle of 4 a.m - 4 p.m (patient 7) or 6 a.m - 6 p.m (patient 1), 21 °C and 50% humidity. At terminal analysis, BM was collected from femur, tibia and crista from both hind legs of all mice.

Model 1: NOD.Cg-*Prkdc^{scid} Il2rg^{tm1Wjl}* Tg(CMV-IL3,CSF2,KITLG)1Eav/MloySzJ (NSG-SGM3) mice stably engrafted with primary FLT3^{D/Y} HLA-A2^{pos} AML cells from patient 7 at 5 weeks of age were obtained from the Jackson Laboratory (Stock ID J000106565). Upon arrival (5.5 weeks after transplantation), PB myeloid engraftment was confirmed by flow cytometry and mice were allocated to treatment groups (TCR^{FLT3D/Y} or TCR^{1G4} cells) based on their engraftment levels to ensure that the mean human myeloid engraftment was similar between the two groups before infusion of T cells. Cryopreserved TCR-transduced T cells were thawed and cultured in X-VIVO^{™} 20 medium (Lonza) supplemented with 5 % human serum, 1% Penicillin/Streptavidin and 5ng/ml IL-7 and IL-15 for 3-4 days prior to treatment. T cells containing 5×10⁶ CD8⁺mTCR-β⁺ T cells were injected through the lateral tail vein, and all mice received daily IP injections of 2500 IU human IL-2 (R&D Systems). At day 8 after T-cell infusion, half of the mice received a second dose of 5×10⁶ CD4-depleted (Miltenyi Biotec) TCR^{FLT3D/Y} or TCR^{1G4} T cells. As no difference was observed between the mice receiving one, or two, doses of T cells in either leukemic engraftment or T cell expansion, the data from these mice were pooled. The effect of the T cells was monitored in serially collected PB, and at termination 15 days after T cell treatment in the BM and spleen through detailed flow cytometry analysis.

Model 2: CD34⁺ cells from patient 1 (FLT3^{D/Y} and HLA-A2^{pos}) were obtained from cryopreserved BM MNCs by CD34 magnetic bead enrichment (Miltenyi Biotec) according to manufacturer's instructions and as previously described (78). 3×10⁵ CD34⁺ cells/mouse were intrafemorally injected into sub-lethally irradiated (3.3 Gy, X-ray source) female and male NSG-SGM3 mice (The Jackson Laboratory, stock 013062) 9 weeks of age. Engraftment was monitored through flow cytometry analysis of PB and upon confirmation of stable myeloid engraftment 7 weeks after transplantation, mice were allocated to treatment groups (TCR^{FLT3D/Y} or TCR^{1G4} cells) based on their engraftment levels as described for patient 7. Males and females were distributed equally into the treatment groups. Cryopreserved T cells were thawed and cultured as described above and T cells containing 5×10⁶ CD8⁺mTCR-β⁺ T cells were infused into each mouse by lateral tail vain injections and all mice received daily IP injections of 2500 IU human IL-2 for 2 weeks followed by less frequent injections. The effect of the T cells was monitored in serially collected PB and at termination 34 days after T cell treatment in the BM and spleen.

Model 3: In order to establish mice with low leukemic engraftment levels and mimic an MRD setting, secondary transplantation of BM cells from patient 1-engrafted NSG mice (The Jackson Laboratory, stock 005557) was performed into sub-lethally irradiated (2.5 Gy, X-ray source) female NSG mice 12-13 weeks of age through intrafemoral injections. Following confirmation of stable engraftment 20 weeks after secondary transplantation, mice were treated with TCR^{FLT3D/Y} or TCR^{1G4} CD4- depleted (Miltenyi Biotec) T cells (10×10⁶ T cells, containing 90 % CD8 and 10 % CD4 T cells). All mice were injected daily with IP injections of 2500 IU human IL-2. The effect of the T cells was evaluated in BM 11 days after T-cell treatment.

Model 4: Following secondary transplantation of BM from mice engrafted with patient 1 cells, BM from three NOD.Cg-*Prkdc^{scid} Il2rg^{tm1Sug}* Tg(CMV-IL2)4-2Jic/JicTac (NOG-hIL2, Taconic) mice was isolated and cultured for 48 h in CellGro GMP DC medium supplemented with 5% human serum, IL-7 and IL-15 either with no T cells or with TCR^{1G4} or TCR^{FLT3D/Y} cells (effector:target cell ratio, 1:2). After 48h, the contents of each well were harvested and intrafemorally injected into sub-lethally irradiated (2.25-2.5 Gy) female NSG mice 8-13 weeks of age. Engraftment levels were monitored in serially collected PB samples by flow cytometry (FACS). As no difference in engraftment levels were observed between donor mice, data were pooled from two individual experiments. Experimental mice were housed 2-5 mice per cage in IVC-Mouse GM 500 cages with a light cycle of 6 a.m - 6 p.m, 21 °C and 45% humidity. Differences in engraftment dynamics between mice engrafted with AML cells pre-cultured with TCR^{FLT3D/Y}, TCR^{1G4} or without T cells was assessed by multilevel linear regression using the R package "lmerTest". Sampling time points and groups are treated as an interaction term with random effects for individual mice. Because the engraftment at the first time point was 0 for all mice except one, we fixed the intercept as 0. Estimated marginal means of models were obtained and compared between 3 groups using the R package "emmeans". Multiple tests were corrected using the Benjamini-Hochberg method.

Whole exome sequencing of AML cells from patient 1 and corresponding PDX mice

Whole bone marrow from AML patient 1, and from one TCR^{1G4} cell treated PDX mouse and one untreated PDX mouse terminated d34 were sorted on a FACSAria Fusion (BD Biosciences). From AML patient 1, primary AML blasts (CD3⁻CD19⁻) and primary T cells (CD3⁺CD8⁺ or CD4⁺CD19⁻CD33⁻) were sorted. From the TCR^{1G4} cell-treated PDX mouse and the untreated PDX mouse AML cells were sorted as mCD45⁻hCD45⁺CD3⁻CD19⁻ events. DNA was isolated by Maxwell RSC Cultured Cell DNA Kit (Promega) and whole-exome sequencing libraries were prepared with Lotus DNA Library Prep Kit (IDT). Exon regions were captured using xGen Exome Hyb Panel v2 and xGen Hybridization and Wash Kit (IDT) on 2022/07/04-2022/07/05. After mixing with 1% of PhiX, prepared libraries were sequenced using NextSeq high output kit (300 cycles) with the setting of Read1: 151, Index 1: 8, Index2: 8, Read2: 151. Sequencing reads were aligned to the human (GRCh37) genome reference using Burrows-Wheeler Aligner version 0.7.17 with default parameter settings. PCR duplicates were marked with biobambam version 2.0.87. Reads were subjected to indel realignment and base quality score recalibration using GATK3 (version 3.8) and recalculation of MD/NM tags using SAMtools version 1.9. Errors associated with enzymatic fragmentation during library preparation were removed using FADE version 0.5.5 resulting in an average depth of 364 (331-439) in the final bam files (79).

Mutations calling was performed using GenomonFisher
(https://github.com/Genomon-Project/GenomonFisher) with the following parameters:
(i) Mapping Quality score ≥ 20
(ii) Base Quality score ≥ 15
(iii) Number of total reads >_ 8
(iv) Number of variant reads ≥ 5
(v) Variant allele frequency >_ 0.05
(v) Variant allele frequency in paired T cells < 0.1
(vi) Variant allele frequency in other non-paired normal controls < 0.05 in all and average < 0.01
(vii) Strand ratio in tumor ≠ 0 or 1
(viii) VAF by base counts / VAF by reads count ≥0.5 and ≤2
(ix) P value by Fisher < 0.1
(x) P value by EBFilter (80) < 0.001
(xi) Mutation on exons
(xiii) Not on the repeat regions

Called mutations were annotated by ANNOVAR (81). Copy number analysis was performed using CNACS (82). The WT1 H507P mutation was together with the FLT3 D835Y mutation selected for further quantification in the treated mice by droplet digital PCR because the WT1 mutation was the only other identified potential driver mutation in AML (15, 83).

Droplet digital PCR of cells from patient-derived AML xenografts

To quantify clonal involvement, droplet digital PCR (ddPCR) was performed on isolated DNA. DNA from AML patient 1 cells were isolated and amplified as explained above (WES). DNA from AML PDX mice were isolated through FACS sorting of mCD45⁻mTer119⁻hCD45⁺CD3⁻ cells (PDX patient 7) or hCD45⁺CD33⁺CD34⁻, hCD45⁺CD33⁺CD34⁺ and hCD45⁺CD19⁺ cells (PDX patient 1), and was subjected to whole genome DNA amplification using REPLLg single cell kit (Qiagen) according to the manufacturer's instructions and used for subsequent ddPCR analysis. Populations with <90 cells sorted for ddPCRs were excluded from the analysis. Briefly, a 20 µl PCR reaction mixture containing 1× ddPCR supermix for probes (no dUTP) (Biorad) 1x primer-probe assay (dHsaMDV2010047, dHsaMDS871718945, Bio-Rad) designed specifically for the FLT3^{D835Y} and WT1^{H507P} mutations respectively, and 60ng of non-amplified DNA or whole genome amplified DNA was prepared and mixed with Droplet Generation Oil for Probes (Bio-Rad). Droplets were prepared according to manufacturer's instructions on a QX200 droplet generator (Bio-Rad). Emulsified PCR reactions were run on a thermal cycler (Bio-Rad) incubating the plates at 95°C for 10 min followed by 40 cycles at 94°C for 30 sec and 55°C for 60 sec, followed by 10 min incubation at 98°C. Plates were read on a QX200 droplet reader (Bio-Rad) and results analyzed using QuantaSoft v1.5.38.1118 software (Bio-Rad). QuantaSoft software was used to calculate Variant Allele Frequencies (VAFs) and their 95% confidential intervals of the mutations based on Poisson distribution as described in Bio-Rad's "Droplet Digital PCR applications guide". The absolute numbers of FLT3^{D/Y} cells in the AML PDX mice were determined by the fraction of mutated cells identified by ddPCR, the frequency of phenotypically defined subsets (hCD45⁺CD3- or hCD45⁺CD33⁺CD34- and hCD45⁺CD33⁺CD34⁺, respectively, for patient 7 and 1) in the BM determined by flow cytometry, and the total number of MNCs in the BM counted with Sysmex.

External AML targeted sequencing data analysis

Mutation data (SNVs and indels) from AML patients reported in Papaemmanuil *et al* (15) were downloaded from www.cBioPortal.org. Variant allele frequency (VAF) was estimated from reported alternative allele reads divided by sequencing depth for the position. Patients harboring a FLT3^{D835Y} mutation were selected for in-depth analysis.

The order of FLT3 mutations in AML using VAF and mutated cell fraction was determined from the publicly available mutation list identified by targeted DNA sequencing in Morita *et al* (44).

### Statistical analysis

Statistical analysis was performed in GraphPad Prism v.6-8 (GraphPad Software). In all studies, data represent biological replicates (*n*) and are depicted as mean ± s.e.m. as indicated in figure legends. Comparison of mean values between two experimental groups was conducted with unpaired, two-tailed Student's test. To compare more than two experimental groups, ordinary one-way analysis of variance (ANOVA) test with adjustment for multiple comparisons with Tukey's post-test was employed. To determine differences between *in vivo* treatment groups in the PDX NSG mouse models, Kruskal-Wallis ANOVA by Dunn's multiple comparisons test and two-tailed Mann-Whitney test were performed. *P* values < 0.05 were considered statistically significant.

## Claims

1. A protein for specific binding to a human leukocyte antigen (HLA) type A2 presenting an HLA-class 1 peptide comprising the sequence YIMSDSNYV (SEQ ID NO: 1),
wherein the protein comprises an α-chain variable domain and a β-chain variable domain, which together form an antigen binding unit;
wherein the α-chain variable domain comprises three complementarity-determining regions (CDRs); CDR1, CDR2 and CDR3 which respectively comprise the amino acid sequences SEQ ID NO: 2, 3 and 4,
and
wherein the β-chain variable domain comprises three CDRs; CDR1, CDR2 and CDR3 which respectively comprise the amino acid sequences SEQ ID NO: 5, 6 and 7.

2. The protein according to claim 1, wherein
the α-chain variable domain comprises the amino acid sequence set forth in SEQ ID NO: 8, or an amino acid sequence having at least 90 % sequence identity thereto; and
the β-chain variable domain comprises the amino acid sequence set forth in SEQ ID NO: 9, or an amino acid sequence having at least 90 % sequence identity thereto.

3. The protein according to claims 1 or 2, wherein the α-chain variable domain is fused to an extracellular α-chain constant domain and the β-chain variable domain is fused to an extracellular β-chain constant domain.

4. The protein according to claim 3, wherein the extracellular α-chain constant domain is represented by SEQ ID NO: 10 and the extracellular β-chain constant domain represented by SEQ ID NO: 11.

5. The protein according to claims 3 or 4, wherein the extracellular α-chain constant domain is fused to a transmembrane domain.

6. The protein according to claims 3 to 4, wherein the extracellular β-chain constant domain is fused to a transmembrane domain.

7. The protein according to claims 5 or 6, wherein each of the extracellular α-chain constant domain and the extracellular β-chain constant domain are fused to a transmembrane domain.

8. The protein according to any one of claims 5 to 7, wherein the transmembrane domain is fused to an intracellular signaling domain.

9. The protein according to any one of claims 1 to 8, wherein the protein comprises a full-length α-chain and/or a full-length β-chain.

10. The protein according to any one of claims 1 to 9, wherein the protein comprises a full-length α-chain represented by SEQ ID NO: 12 and/or a full-length β-chain represented by SEQ ID NO: 13, or alternatively, a protein according to claim 1 comprising a full-length α-chain represented by SEQ ID NO: 14 and/or a full-length β-chain represented by SEQ ID NO: 15.

11. One or more recombinant nucleic acid molecules which alone or together encode a protein according to any one of claims 1 to 10.

12. One or more cDNA molecules encoding the protein according to any one of claims 1 to 10.

13. An immune effector cell transduced with a nucleic acid according to claims 11 or 12.

14. An immune effector cell expressing a protein according to any one of claims 1 to 10 in its cell membrane.

15. An immune effector cell according to claims 13 or 14, wherein said cell is a T-helper cell, a cytotoxic T-cell or a natural killer cell.

16. A pharmaceutical composition comprising the proteins according to any one of claim 1 to 4, the nucleic acid according to claims 11 or 12, or a cell according to claims 13 or 4.

17. A pharmaceutical composition for intravenous administration comprising T-cells expressing a protein according to any one of claims 1 to 10 in their cell membrane.

18. A method for treatment of acute myeloid leukemia (AML) comprising the steps of
a) providing a biological sample comprising cancer cells from a subject diagnosed with AML,
b) performing an assay for detecting the D835Y mutation in FLT3 in the biological sample,
c) performing HLA-typing of the subject, and
d) administering the pharmaceutical composition as defined in claim 17 via intravenous infusion to the subject if the assay in b) is positive and the subject is HLA-A*02:01 positive.
